# FASCICULE DE BREVET EUROPEEN

(11) **EP 0 713 922 B1**
(45) Date de publication et mention de la délivrance du brevet: **08.09.1999**
(21) Numéro de dépôt: 95402411.3
(22) Date de dépôt: 27.10.1995
(51) Int. Cl.: C12Q 1/68, C12P 19/34, C07H 21/04

(54) **Oligonucléotide utilisable comme amorce dans une méthode d'amplification basée sur une réplication avec déplacement de brin**
Oligonukleotid verwendbar als Primer in einem Amplifikationsverfahren auf der Basis einer Strangverdrängungsreplikation
Oligonucleotide usable as a primer in a method of amplification based on a replication with strand displacement

(30) Priorité: 28.10.1994 FR 9413010
(43) Date de publication de la demande: 29.05.1996
(73) Titulaire: BIO MERIEUX, Société anonyme, F-69280 Marcy l'Etoile (FR)
(72) Inventeur: Cleuziat, Philippe, F-69003 Lyon (FR); Guillou-Bonnici, Françoise, F-69100 Villeurbanne (FR); Levasseur, Pierre, Watertown, M.A. 02172 (US); Mallet, François, F-69100 Villeurbanne (FR)
(74) Mandataire: Tonnellier, Jean-Claude

(56) Documents cités:
- EP-A- 0 395 398
- EP-A- 0 427 073
- EP-A- 0 530 112
- EP-A- 0 543 612
- WO-A-93/05184
- BIOTECHNOLOGY, vol. 12, Juin 1994 pages 619-623, CAETANO ET AL 'dna amplification fingerprinting using arbitrary mini-hairpin primers'

### LISTE DE SEQUENCES

(1) INFORMATIONS GENERALES:
   (i) DEPOSANT:
      (A) NOM: BIO MERIEUX
      (B) RUE: Chemin de l'Orme
      (C) VILLE: MARCY L'ETOILE
      (E) PAYS: FRANCE
      (F) CODE POSTAL: 69280
      (G) TELEPHONE: 78872000
      (H) TELECOPIE: 78872090
   (ii) TITRE DE L' INVENTION: Oligonucléotide utilisable comme amorce dans une méthode d'amplification basée sur une réplication avec déplacement de brin.
   (iii) NOMBRE DE SEQUENCES: 43
   (iv) FORME DECHIFFRABLE PAR ORDINATEUR:
      (A) TYPE DE SUPPORT: Disquette 3 ½
      (B) ORDINATEUR: PC compatible
      (C) SYSTEME D' EXPLOITATION: PC-DOS/MS-DOS
      (D) LOGICIEL: MSWORD 6.0 pour MS-DOS 6.20 / WINDOWS 3.10
   (v) DONNEES DE LA DEMANDE ACTUELLE:
      NUMERO DE LA DEMANDE: EP 95 402411.3
   (vi) DONNEES DE LA DEMANDE ANTERIEURE:
      (A) NUMERO DE LA DEMANDE: FR 94 13010
      (B) DATE DE DEPOT: 28-OCT-1994
(2) INFORMATIONS POUR LA SEQ ID NO: 1:
   (i) CARACTERISTIQUES DE LA SEQUENCE:
      (A) LONGUEUR: 76
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N = résidu 3-amino-1,2-propane-diol
   (xi) DESCRIPTION DE LA SEQUENCE: SEQ ID NO: 1:
(2) INFORMATIONS POUR LA SEQ ID NO: 2:
   (i) CARACTERISTIQUES DE LA SEQUENCE:
      (A) LONGUEUR: 75
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu hexaéthyléneglycol
      (xi) DESCRIPTION DE LA SEQUENCE: SEQ ID NO: 2:
   TCCATCGCTT CGGGNAACGA AGCGATGGAA GTAATTTAAT ACGACTCACT ATAGAAAGAG 60
   GATGGCATGA CAGTA 75
(2) INFORMATIONS POUR LA SEQ ID NO: 3:
   (i) CARACTERISTIQUES DE LA SEQUENCE:
      (A) LONGUEUR: 77
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N = résidu 3-amino-1.2-propane-diol
   (xi) DESCRIPTION DE LA SEQUENCE: SEQ ID NO: 3:
   TCCATCGCTT CGGGNNAACG AAGCGATGGA AGTAATTTAA TACGACTCAC TATAGAAAGA 60
   GTTGGCCGCA GTGTTAT 77
(2) INFORMATIONS POUR LA SEQ ID NO: 4:
   (i) CARACTERISTIQUES DE LA SEQUENCE:
      (A) LONGUEUR: 76
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu hexaéthylèneglycol
   (xi) DESCRIPTION DE LA SEQUENCE: SEQ ID NO: 4:
   TCCATCGCTT CGGGNAACGA AGCGATGGAA GTAATTTAAT ACGACTCACT ATAGAAAGAG 60
   TTGGCCGCAG TGTTAT 76
(2) INFORMATIONS POUR LA SEQ ID NO: 5:
   (i) CARACTERISTIQUES DE LA SEQUENCE:
      (A) LONGUEUR: 46
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu 3-amino-1,2-propane-diol
   (xi) DESCRIPTION DE LA SEQUENCE:SEQ ID NO: 5:
   TCCATCGCTT CGGGNNAACG AAGCGATGGA GGATGGCATG ACAGTA 46
(2) INFORMATIONS POUR LA SEQ ID NO: 6:
   (i) CARACTERISTIQUES DE LA SEQUENCE:
      (A) LONGUEUR: 45
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu hexaéthylèneglycol
   (xi) DESCRIPTION DE LA SEQUENCE: SEQ ID NO: 6:
   TCCATCGCTT CGGGNAACGA AGCGATGGAG GATGGCATGA CAGTA 45
(2) INFORMATIONS POUR LA SEQ ID NO: 7:
   (i) CARACTERISTIQUES DE LA SEQUENCE:
      (A) LONGUEUR: 47
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu 3-amino-1,2-propane-diol
   (xi) DESCRIPTION DE LA SEQUENCE: SEQ ID NO: 7:
   TCCATCGCTT CGGGNNAACG AAGCGATGGA GTTGGCCGCA GTGTTAT 47
(2) INFORMATIONS POUR LA SEQ ID NO: 8:
   (i) CARACTERISTIQUES DE LA SEQUENCE:
      (A) LONGUEUR: 46
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (ix) CARACTERISTIQUE:
      (D) AUTRES INFORMATIONS:
         N = résidu hexaéthylèneglycol
   (xi) DESCRIPTION DE LA SEQUENCE: SEQ ID NO: 8:
   TCCATCGCTT CGGGNAACGA AGCGATGGAG TTGGCCGCAG TGTTAT 46
(2) INFORMATIONS POUR LA SEQ ID NO: 9:
   (i) CARACTERISTIQUES DE LA SEQUENCE:
      (A) LONGUEUR: 861
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 9:
   ATGAGTATTC AACATTTCCG TGTCGCCCTT ATTCCCTTTT TTGCGGCATT TTGCCTTCCT 60
   GTTTTTGCTC ACCCAGAAAC GCTGGTGAAA GTAAAAGATG CTGAAGATCA GTTGGGTGCA 120
   CGAGTGGGTT ACATCGAACT GGATCTCAAC AGCGGTAAGA TCCTTGAGAG TTTTCGCCCC 180
   GAAGAACGTT TTCCAATGAT GAGCACTTTT AAAGTTCTGC TATGTGGCGC GGTATTATCC 240
   CGTGTTGACG CCGGGCAAGA GCAACTCGGT CGCCGCATAC ACTATTCTCA GAATGACTTG 300
   GTTGAGTACT CACCAGTCAC AGAAAAGCAT CTTACGGATG GCATGACAGT AAGAGAATTA 360
   TGCAGTGCTG CCATAACCAT GAGTGATAAC ACTGCGGCCA ACTTACTTCT GACAACGATC 420
   GGAGGACCGA AGGAGCTAAC CGCTTTTTTG CACAACATGG GGGATCATGT AACTCGCCTT 480
   GATCGTTGGG AACCGGAGCT GAATGAAGCC ATACCAAACG ACGAGCGTGA CACCACGATG 540
   CCTGCAGCAA TGGCAACAAC GTTGCGCAAA CTATTAACTG GCGAACTACT TACTCTAGCT 600
   TCCCGGCAAC AATTAATAGA CTGGATGGAG GCGGATAAAG TTGCAGGACC ACTTCTGCGC 660
   TCGGCCCTTC CGGCTGGCTG GTTTATTGCT GATAAATCTG GAGCCGGTGA GCGTGGGTCT 720
   CGCGGTATCA TTGCAGCACT GGGGCCAGAT GGTAAGCCCT CCCGTATCGT AGTTATCTAC 780
   ACGACGGGGA GTCAGGCAAC TATGGATGAA CGAAATAGAC AGATCGCTGA GATAGGTGCC 840
   TCACTGATTA AGCATTGGTA A 861
(2) INFORMATIONS POUR LA SEQ ID NO: 10:
   (i) CARACTERISTIQUES DE LA SEQUENCE:
      (A) LONGUEUR: 45
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 10:
   AATTCTAATA CGACTCACTA TAGGGAGATT ACCAATGCTT AATCA 45
(2) INFORMATIONS POUR LA SEQ ID NO: 11:
   (i) CARACTERISTIQUES DE LA SEQUENCE:
      (A) LONGUEUR: 18
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 11:
   ATGAGTATTC AACATTTC 18
(2) INFORMATIONS POUR LA SEQ ID NO: 12:
   (i) CARACTERISTIQUES DE LA SEQUENCE:
      (A) LONGUEUR: 100
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 12:
   GTTGGCCGCA GTGTTACTAC TCATGGTTAT GGCAGCACTG CATAATTCTC TTACTGTCAT 60
   GCCATCCGTA AGATGCTTTT CTGTGACTGG TGAGTACTCA 100
(2) INFORMATIONS POUR LA SEQ ID NO: 13:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 13:
   AGTACTCACC AGTCACA 17
(2) INFORMATIONS POUR LA SEQ ID NO: 14:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 14:
   GCACTGCATA ATTCTCT 17
(2) INFORMATIONS POUR LA SEQ ID NO: 15:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 15:
   CACTCATGGT TATGGCA 17
(2) INFORMATIONS POUR LA SEQ ID NO: 16:
   (i) CARACTERISTIQUES DE LA SEQUENCE:
      (A) LONGUEUR: 18
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 16:
   AATTAACCCT CACTAAAG 18
(2) INFORMATIONS POUR LA SEQ ID NO: 17:
   (i) CARACTERISTIQUES DE LA SEQUENCE:
      (A) LONGUEUR: 45
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 17:
   AATTCTAATA CGACTCACTA TAGGGAGACC CCGAAGAACG TTTTC 45
(2) INFORMATIONS POUR LA SEQ ID NO: 18:
   (i) CARACTERISTIQUES DE LA SEQUENCE:
      (A) LONGUEUR: 19
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 18:
   CTCCTTCGGT CCTCCGATC 19
(2) INFORMATIONS POUR LA SEQ ID NO: 19:
   (i) CARACTERISTIQUES DE LA SEQUENCE:
      (A) LONGUEUR: 18
      (B) TYPE: acide nucléique
      (C) NOMBRE DE BRINS: simple
      (D) CONFIGURATION: linéaire
   (ii) TYPE DE MOLECULE: ADN génomique
   (iii) HYPOTHETIQUE: NON
   (iv) ANTI-SENS: NON
   (xi) DESCRIPTION DE LA SEQUENCE: SEQ ID NO: 19:
   TAATACGACT CACTATAG 18
(2) INFORMATIONS POUR LA SEQ ID NO: 20:
   (i) CARACTERISTIQUES DE LA SEQUENCE:
      (A) LONGUEUR: 18
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 20:
   ATTTAGGTGA CACTATAG 18
(2) INFORMATIONS POUR LA SEQ ID NO: 21:
   (i) CARACTERISTIQUES DE LA SEQUENCE:
      (A) LONGUEUR: 58
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 21:
   NATANATANN NNCATTTNNT AATACGACTC ACTATAGAAA GAGGATGGCA TGACAGTA 58
(2) INFORMATIONS POUR LA SEQ ID NO: 22:
   (i) CARACTERISTIQUES DE LA SEQUENCE:
      (A) LONGUEUR: 16
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 22:
   TACTGTCATG CCATCC 16
(2) INFORMATIONS POUR LA SEQ ID NO: 23:
   (i) CARACTERISTIQUES DE LA SEQUENCE:
      (A) LONGUEUR: 66
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 23:
   TCTAATCCTG TTTGCTCCCC AGTAATTTAA TACGACTCAC TATAGAAAGA GGATGGCATG 60
   ACAGTA 66
(2) INFORMATIONS POUR LA SEQ ID NO: 24:
   (i) CARACTERISTIQUES DE LA SEQUENCE:
      (A) LONGUEUR: 67
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 24:
   TCTAATCCTG TTTGCTCCCC AGTAATTTAA TACGACTCAC TATAGAAAGA GTTGGCCGCA 60
   GTGTTAT 67
(2) INFORMATIONS POUR LA SEQ ID NO: 25:
   (i) CARACTERISTIQUES DE LA SEQUENCE:
      (A) LONGUEUR: 20
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 25:
   TCTAATCCTG TTTGCTCCCC 20
(2) INFORMATIONS POUR LA SEQ ID NO: 26:
   (i) CARACTERISTIQUES DE LA SEQUENCE:
      (A) LONGUEUR: 14
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 26:
   AACGAAGCGA TGGA 14
(2) INFORMATIONS POUR LA SEQ ID NO: 27:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 27:
   AGAGAATTAT GCAGTGC 17
(2) INFORMATIONS POUR LA SEQ ID NO: 28:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 28:
   TGCCATAACC ATGAGTG 17
(2) INFORMATIONS POUR LA SEQ ID NO: 29:
   (i) CARACTERISTIQUES DE LA SEQUENCE:
      (A) LONGUEUR: 54
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         T positions 16 à 19 = α thymidine
   (xi) DESCRIPTION DE LA SEQUENCE: SEQ ID NO: 29:
   CCAACCACTC ACTCCTTTTG GAGTGAGTGG TTGGTTACGG ATGGCATGAC AGTA 54
(2) INFORMATIONS POUR LA SEQ ID NO: 30:
   (i) CARACTERISTIQUES DE LA SEQUENCE:
      (A) LONGUEUR: 44
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N = résidu 3-amino-1,2-propane-diol
   (xi) DESCRIPTION DE LA SEQUENCE: SEQ ID NO: 30:
   ATGACAGTAC GGTAGGAGGT AGCGAAGCTT TTNCTTCGCT ACCT 44
(2) INFORMATIONS POUR LA SEQ ID NO: 31:
   (i) CARACTERISTIQUES DE LA SEQUENCE:
      (A) LONGUEUR: 51
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS :
         N = résidu hexaéthylèneglycol
   (si) DESCRIPTION DE LA SEQUENCE: SEQ ID NO: 31:
(2) INFORMATIONS POUR LA SEQ ID NO: 32:
   (i) CARACTERISTIQUES DE LA SEQUENCE:
      (A) LONGUEUR: 62
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N = résidu 3-amino-1,2-propane-diol
   (xi) DESCRIPTION DE LA SEQUENCE: SEQ ID NO: 32:
   CCAACCACTC ACTCCGCCGG TAANGGCGGA GTGAGTGGTT GGTTACGGAT GGCATGACAG 60
   TA 62
(2) INFORMATIONS POUR LA SEQ ID NO: 33:
   (i) CARACTERISTIQUES DE LA SEQUENCE:
      (A) LONGUEUR: 54
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 33:
   CCAACCACTC ACTCCTTTTG GAGTGAGTGG TTGGTTACGG ATGGCATGAC AGTA 54
(2) INFORMATIONS POUR LA SEQ ID NO: 34:
   (i) CARACTERISTIQUES DE LA SEQUENCE:
      (A) LONGUEUR: 52
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N en position 1 = 6-chloro-2-methoxyacridine
         N en position 17 = résidu hexaéthylèneglycol
   (xi) DESCRIPTION DE LA SEQUENCE: SEQ ID NO: 34:
   NCCAACCACT CACTCCNGGA GTGAGTGGTT GGTTACGGAT GGCATGACAG TA 52
(2) INFORMATIONS POUR LA SEQ ID NO: 35:
   (i) CARACTERISTIQUES DE LA SEQUENCE:
      (A) LONGUEUR: 55
      (B) TYPE: acide nucléique
      (C) NOMBRE DE BRINS: simple
      (D) CONFIGURATION: linéaire
   (ii) TYPE DE MOLECULE: ADN génomique
   (ix) CARACTERISTIQUE:
      (D) AUTRES INFORMATIONS:
         N = 6 chloro-2-méthoxyacridine
   (xi) DESCRIPTION DE LA SEQUENCE: SEQ ID NO: 35:
   NCCAACCACT CACTCCTTTT GGAGTGAGTG GTTGGTTACG GATGGCATGA CAGTA 55
(2) INFORMATIONS POUR LA SEQ ID NO: 36:
   (i) CARACTERISTIQUES DE LA SEQUENCE:
      (A) LONGUEUR: 43
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 36:
   TAATACGACT CACTATAGGG AGACTCCTTC GGTCCTCCGA TCG 43
(2) INFORMATIONS POUR LA SEQ ID NO: 37:
   (i) CARACTERISTIQUES DE LA SEQUENCE:
      (A) LONGUEUR: 45
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 37:
   AATTCTAATA CGACTCACTA TAGGGAGATT ACCAATGCTT AATCA 45
(2) INFORMATIONS POUR LA SEQ ID NO: 38:
   (i) CARACTERISTIQUES DE LA SEQUENCE:
      (A) LONGUEUR: 17
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 38:
   GGATGGCATG GACAGTA 17
(2) INFORMATIONS POUR LA SEQ ID NO: 39:
   (i) CARACTERISTIQUES DE LA SEQUENCE:
      (A) LONGUEUR: 40
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 39:
   TAATACGACT CACTATAGAA AGAGTTGGCC GCAGTGTTAT 40
(2) INFORMATIONS POUR LA SEQ ID NO: 40:
   (i) CARACTERISTIQUES DE LA SEQUENCE:
      (A) LONGUEUR: 35
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 40:
   GGAGTGAGTG GTTGGTTACG GATGGCATGA CAGTA 35
(2) INFORMATIONS POUR LA SEQ ID NO: 41:
   (i) CARACTERISTIQUES DE LA SEQUENCE:
      (A) LONGUEUR: 13
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 41:
   GGAGTGAGTG GTT 13
(2) INFORMATIONS POUR LA SEQ ID NO: 42:
   (i) CARACTERISTIQUES DE LA SEQUENCE:
      (A) LONGUEUR: 12
      (B) TYPE: acide nucléique
      (C) NOMBRE DE BRINS: simple
      (D) CONFIGURATION: linéaire
   (ii) TYPE DE MOLECULE: ADN génomique
   (xi) DESCRIPTION DE LA SEQUENCE: SEQ ID NO: 42:
   AACGAAGCGA TG 12
(2) INFORMATIONS POUR LA SEQ ID NO: 43:
   (i) CARACTERISTIQUES DE LA SEQUENCE:
      (A) LONGUEUR: 20
      (B) TYPE: acide nucléique
      (C) NOMBRE DE BRINS: simple
      (D) CONFIGURATION: linéaire
   (ii) TYPE DE MOLECULE: ADN genomique
   (xi) DESCRIPTION DE LA SEQUENCE: SEQ ID NO: 43:
   TTGGTATGGC TTCATTCAGC 20

## Description

La présente invention concerne des oligonucléotides capables de former des structures de type tige-boucle présentant la propriété de ne pas être recopiables en totalité par une ADN ou une ARN polymérase et pouvant servir d'amorces de réplication pour ces mêmes enzymes. L'invention concerne également un procédé d'amplification d'acides nucléiques à l'aide de telles amorces.

Il est souvent nécessaire, dans les technologies relatives aux acides nucléiques et au matériel génétique, de déterminer si un gène, une partie de gène ou une séquence nucléotidique est présent chez un organisme vivant, dans un extrait cellulaire de cet organisme ou dans un échantillon biologique.

L'intérêt de la recherche de séquences nucléotidiques spécifiques est immense, notamment pour la détection d'organismes pathogènes, la détermination de la présence d'allèles, la détection de la présence de lésions dans un génome hôte et la détection de la présence d'un ARNm particulier ou de la modification d'un hôte cellulaire. Les maladies génétiques telles que la maladie de Huntington, la myopathie de Duchenne, la phénylcétonurie et la β-thalassémie peuvent être diagnostiquées par le biais de l'analyse de l'ADN des individus. De plus, le diagnostic ou l'identification des virus, viroïdes, bactéries, champignons, protozoaires ou quelque autre forme de vie végétale ou animale peut être réalisé par des expériences d'hybridation avec des sondes nucléiques.

Dans les quelques exemples cités précédemment, après avoir identifié une séquence spécifique d'un organisme ou d'une maladie, il convient d'extraire les acides nucléiques d'un échantillon, et de déterminer si cette séquence est présente. De nombreuses méthodes de détection ont été développées dans ce but. Ces méthodes, et particulièrement celles qui requièrent la détection de polynucléotides, reposent sur les propriétés d'appariement purine-pyrimidine des brins complémentaires d'acides nucléiques dans les duplex ADN-ADN, ADN-ARN et ARN-ARN. Ce processus d'appariement s'effectue par l'établissement de liaisons hydrogène entre les bases adénine-thymine (A-T) et guanine-cytosine (G-C) de l'ADN double brin ; des paires de bases adénine-uracile (A-U) peuvent également se former par liaison hydrogène dans les duplex ADN-ARN ou ARN-ARN. L'appariement de brins d'acides nucléiques pour la détermination de la présence ou de l'absence d'une molécule d'acide nucléique donnée est communément appelée "hybridation d'acides nucléiques" ou simplement "hybridation".

Pour la mise en oeuvre de l'invention, il est généralement nécessaire qu'une ou plusieurs séquences spécifiques de la cible aient été préalablement identifiées. La méthode la plus directe pour détecter la présence d'une séquence cible dans un échantillon d'acides nucléiques est d'obtenir une "sonde" dont la séquence est suffisamment complémentaire d'une partie de l'acide nucléique cible pour s'hybrider à celui-ci. La sonde ainsi synthétisée peut être appliquée dans un échantillon contenant des acides nucléiques, et si la séquence cible est présente, la sonde s'hybridera pour former un produit de réaction. En l'absence de séquence cible et en évitant tout phénomène d'hybridation non spécifique, aucun produit de réaction ne sera formé. Si la sonde synthétisée est couplée à un marqueur détectable, le produit de réaction peut être détecté en mesurant la quantité de marqueur présent. Les transferts de type Southern (Southern E.M., 1975. *J. Mol. Biol.,* 98 : 503), ou Northern, ou la technique du Dot-Blot ou l'hybridation sandwich (Dunn, A.R. et Hassel, J.A., 1977. *Cell*, 12 : 23) constituent des exemples de méthodes utilisables.

La principale difficulté de cette approche est, cependant, qu'elle n'est pas directement applicable aux cas où le nombre de copies de la séquence cible présentes dans un échantillon est faible. Il est alors difficile de distinguer un signal significatif, c'est-à-dire de distinguer la fixation spécifique d'une sonde sur sa séquence cible de la fixation non spécifique de la sonde sur une séquence différente de la séquence cible. Une des solutions à ce problème consiste à augmenter le signal de détection par une réaction supplémentaire visant à multiplier préalablement de manière spécifique le nombre de copies d'un fragment d'acide nucléique, dit séquence cible, présent dans un échantillon. Cette technique est couramment appelée « amplification ».

Plusieurs techniques d'amplification de cible sont décrites dans la littérature. Elles reposent principalement soit sur la répétition de cycles de synthèse d'ADN par élongation d'amorces nucléotidiques hybridées sur la séquence cible à amplifier par une ADN polymérase ("Polymerase Chain Reaction", dite PCR, brevets des États Unis d'Amérique n°4 683 195, 4 683 202 et 4 800 159; demande de brevet Européen n°0 201 184 ; "Ligase Chain Reaction", dite LCR, demande de brevet Européen n° 0 320 308 ; "Repair Chain Reaction ", dite RCR, demande de brevet international n° WO 90/01069), soit sur la répétition de cycles de synthèse d'ARN *in vitro*, par réaction de transcription par une ARN polymérase ADN ou ARN dépendante dont l'activité est obligatoirement associée à une région spécifique dite région promotrice, renfermant une séquence ou une structure jouant le rôle de promoteur (technique dite TAS, décrite dans la demande de brevet international n°WO88/10315 ; "Self-Sustained Sequence Replication", dite 3SR, décrite dans la demande de brevet international n° WO 90/06995 et la demande de brevet Européen n°0 373 960 ; "Nucleic Acid Sequence-Based Amplification", dite NASBA, décrite dans la demande de brevet international n°WO91/02818 et la demande de brevet Européen n°0 329 822 ; "Single Primer Sequence Replication", dite SPSR, décrite dans le brevet des États Unis d'Amérique n° 5 194 370 et "Ligation Activated Transcription", dite LAT, décrite dans le brevet des États Unis d'Amérique n° 5 194 370).

Néanmoins, toutes ces techniques d'amplification possèdent au moins un inconvénient important. Dans certains cas (PCR, LCR ou RCR), l'inconvénient le plus important est la nécessité de réaliser de nombreux cycles de température afin de dissocier les produits de réaction de la cible. La réalisation de tels cycles successifs de température constitue un inconvénient pour l'automatisation de ces techniques et augmente considérablement le temps réactionnel nécessaire à l'amplification. Un autre inconvénient de certaines techniques d'amplification est la limitation de la taille du produit de réaction d'amplification. Les techniques telles que la RCR ou la LCR ne permettent d'amplifier que la séquence de la cible correspondant aux amorces et aux sondes nucléotidiques utilisées dans le processus d'amplification. Le bruit de fond non spécifique (c'est à dire en absence de la cible) est également un sérieux inconvénient de techniques telles que la LCR. Un autre inconvénient majeur de certaines techniques d'amplification réside dans le nombre élevé d'activités enzymatiques impliquées dans le processus d'amplification. Les méthodes dérivées de la TAS, telles que la 3SR ou la NASBA requièrent au moins quatre activités enzymatiques (ADN polymérase ADN-dépendante, ADN polymérase ARN-dépendante, ARN polymérase ADN dépendante, RNase H), voire cinq dans le cas de la LAT qui utilise en plus une ADN ligase. Il est par conséquent très difficile de rendre ces techniques performantes du fait de la difficulté de parvenir à des conditions réactionnelles satisfaisant simultanément ces quatre ou cinq activités enzymatiques. Enfin, si diverses techniques d'amplification ont recours à des activités de nucléases (exonucléase, endonucléase, RNase) comme moyen de séparation de brins d'acides nucléiques (demande de brevet Européen n° 0 500 224), leur utilisation est néanmoins préjudiciable, en raison du risque de dégradation de la cible ou du produit de réaction par des activités parasites parfois présentes dans des préparations de nucléases. En conséquence, l'activité de ces nucléases doit être rigoureusement dosée, afin de maintenir un équilibre entre l'action des différentes enzymes impliquées. Au vu de ces nombreuses limitations, d'autres méthodes d'amplification alternatives à ces méthodes ont été proposées.

La méthode "Strand Displacement Amplification", dite SDA, décrite dans le brevet des États Unis d'Amérique n° 5 270 184, permet une multiplication isotherme (37°C) d'une séquence d'ADN cible à l'aide d'une enzyme de restriction appropriée et d'une ADN polymérase ADN-dépendante dépourvue d'activité exonucléase (Walker *et al.,* 1992. *Proc. Natl. Acad. Sci. USA* 89 : 392-396). Elle est basée sur l'hybridation d'amorces oligonucléotidiques contenant une séquence de reconnaissance d'une enzyme de restriction à leur extrémité 5'. On procède à une élongation de ces amorces par l'ADN polymérase en présence d'au moins un nucléotide modifié (5'[-thio]dNTP). La digestion de l'ADN par l'endonucléase de restriction provoque la coupure du brin correspondant à l'amorce, laissant intact le brin complémentaire modifié. L'ADN polymérase peut alors réaliser l'extension des amorces générées et libère un brin d'ADN dans le milieu réactionnel grâce à la propriété de déplacement de brin de l'ADN polymérase. Ce brin libéré peut fixer une nouvelle amorce contenant une séquence de fixation d'enzyme de restriction et le cycle peut recommencer. Une méthode similaire à la SDA utilisant une activité exonucléase au lieu d'une endonucléase, appelée "exonuclease-mediated strand displacement amplification", est décrite dans la demande de brevet Européen n° 0 500 224.

Cependant, ces méthodes présentent elles aussi des inconvénients affectant leur efficacité. Ainsi, elles sont limitées par le type de séquence cible à amplifier, puisque cette séquence ne doit pas comporter de site de restriction correspondant à l'endonucléase utilisée dans le procédé. Il est donc indispensable de connaître, sinon la séquence nucléique du fragment à amplifier, au moins la carte de restriction dudit fragment. En outre, cet inconvénient est accru par le choix des endonucléases de restriction qui est restreint à celles ayant la capacité de cliver un site de reconnaissance hémiphosphorothioate et plus généralement des sites comportant des nucléotides modifiés. Outre les inconvénients liés à la synthèse chimique des nucléotides modifiés, ce procédé d'amplification a un rendement limité en raison de la faible efficacité de l'incorporation enzymatique des nucléotides modifiés.

Une autre technique d'amplification isotherme utilisant, comme la PCR, deux amorces et une ADN polymérase a été décrite (brevet des États Unis d'Amérique n°5 223 414). Cette technique utilise la protéine Rec A pour complexer une première amorce nucléotidique à une extrémité 3' d'un brin d'acide nucléique et une seconde amorce à l'extrémité 3' du brin complémentaire de cet acide nucléique, en présence d'ATP. Les complexes ainsi réalisés sont ensuite mis en présence d'une ADN polymérase et de désoxyribonucléosides triphosphates. Plusieurs cycles d'amplification sont ainsi réalisables dans des conditions isothermes. Toutefois, le rendement d'amplification obtenu est plus faible que celui de la PCR car l'étape de séparation des brins complémentaires d'acides nucléiques fait appel au processus de recombinaison qui est plus lent et plus aléatoire que la dénaturation thermique. De plus, cette méthode repose sur l'utilisation d'une protéine (Rec A) conjointement à une polymérase, ce qui rend difficile son optimisation.

Certaines techniques d'amplification font intervenir des amorces ou des sondes nucléiques présentant une structure particulière. Ainsi, dans le cas de la méthode dite LAT (brevet des États Unis d'Amérique n° 5 194 370), l'amplification des séquences cibles, basée sur la transcription, repose sur l'installation par ligation d'une structure tige-boucle de nature ADN comprenant, au sein de la séquence formant la tige, un promoteur fonctionnel pour une ARN polymérase. En outre, l'extrémité 3' de l'oligonucléotide portant la structure tige-boucle est bloquée et ne peut par conséquent servir d'amorce pour une ADN polymérase. D'autres travaux décrivent une utilisation similaire d'une séquence promotrice contenue dans une structure de type tige-boucle (demandes de brevet Européen n° 0 451 591, 0 534 345, demande de brevet Allemand n°42 13 029, Carpenter *et al.,* 1993. *Clin. Chem.* **39** : 1934-1938, Krupp et Söll, 1987,. *FEBS Lett.* **2** : 271-275). La demande de brevet PCT n° WO 93/17127 décrit l'utilisation d'oligonucléotides de nature ADN formant une structure tige-boucle, ligués aux extrémités de la cible ADN, dans un procédé d'amplification de type PCR à l'aide d'une seule amorce. La demande de brevet Européen n°0 530 112 décrit une technique d'amplification mettant en jeu la collaboration d'une origine de réplication et d'une séquence répétée inversée susceptible de former une structure tige-boucle. L'utilisation d'amorces contenant une ministructure tige-boucle a également été décrite par Caetano et Gresshoff (1994, *Bio/Technolgy* 12: 619-623). L'utilisation de ces amorces arbitrairement définies, mais présentant une mini tige-boucle, couplée à la technique d'amplification PCR, permet d'obtenir un grand nombre de fragments d'acide nucléique amplifiés qui sont de tailles hétérogènes et qui nécessitent une étape de séparation électrophorétique afin de réaliser des empreintes génétiques.

Les différents procédés décrits ci-dessus mettent en jeu des oligonucléotides particuliers pouvant présenter une structure tige-boucle, uniquement dans le but soit d'installer en une seule étape un promoteur fonctionnel en amont de la séquence à amplifier, soit de permettre l'hybridation d'amorces spécifiques dans la boucle localisée en amont de la séquence cible puis l'amplification par élongation d'amorce comme dans le cas de la PCR. Ainsi, aucune de ces techniques d'amplification ne permet d'échapper aux divers inconvénients des autres techniques mentionnées plus haut.

Compte tenu de l'analyse précédente, la conception de nouvelles méthodes d'amplification apparaît souhaitable, et la présente invention a notamment pour objet une méthode d'amplification cyclique d'une séquence nucléotidique cible, pouvant fonctionner de manière isotherme, et présentant l'avantage de n'utiliser qu'une polymérase d'acide nucléique en présence de nucléosides triphosphates.

Dans la présente demande, l'expression "amont" désigne une région située du côté de l'extrémité 5' de l'acide nucléique ou de la séquence polynucléotidique dont il s'agit, et l'expression "aval" désigne une région située du côté de l'extrémité 3' dudit acide nucléique ou de ladite séquence polynucléotidique.

Par séquence homologue d'une autre séquence, on désigne une séquence capable de s'hybrider avec une séquence strictement complémentaire de ladite autre séquence.

Par molécule d'acide nucléique "auto-appariée", on désigne une molécule dont au moins une séquence nucléique qui la compose est hybridée avec une séquence nucléique de la même molécule d'acide nucléique ; on parle alors d'auto-appariements ou d'appariements intramoléculaires par opposition aux appariements intermoléculaires qui mettent en jeu deux molécules d'acide nucléique distinctes.

Le terme "nucléosides triphosphates" (NTP) désigne indifféremment des désoxyribonucléosides triphosphates (dNTP) et/ou des ribonucléosides triphosphates (rNTP).

Les termes "fragment d'acide nucléique", "segment d'acide nucléique", "région d'acide nucléique" ou "oligonucléotide" tels qu'utilisés dans la présente demande, désignent un fragment d'ADN ou d'ARN naturel, un polynucléotide naturel ou de synthèse, pouvant contenir éventuellement au moins une base modifiée telle que l'inosine, la méthyl-5-désoxycytidine, la diméthylamino-5-désoxyuridine, la désoxyuridine, la diamino-2,6-purine, la bromo-5-désoxyuridine, la pseudouridine, la pseudoisocytidine ou toute autre base modifiée permettant l'hybridation. Ce polynucléotide peut aussi être modifié au niveau de la liaison internucléotidique (comme par exemple les liaisons phosphorothioate, H-phosphonate, alkyl phosphonate), au niveau du squelette comme c'est le cas par exemple pour les alpha-oligonucléotides (brevet français n° 2 607 507) ou les PNA (Egholm *et al.,* 1992. *J. Am. Chem. Soc.* 114 : 1895-1897). Dans un polynucléotide modifié, plusieurs des modifications qui viennent d'être mentionnées peuvent être présentes en combinaison.

Le terme "séquence nucléique" désigne un enchainement précis de nucléotides, modifiés ou non, permettant de définir un fragment, un segment ou une région d'acide nucléique tels que définis ci-dessus.

Le terme "amorce" désigne une molécule comprenant une structure oligonucléotidique simple brin composée d'au moins cinq nucléotides. Ces nucléotides peuvent être des désoxyribonucléotides et/ou des ribonucléotides. Ces nucléotides peuvent être modifiés comme décrit précédemment dans le paragraphe relatif à la description du terme "fragment d'acide nucléique". Les amorces oligonucléotidiques, une fois hybridées sur une séquence d'acide nucléique (ADN, ARN, ou molécule chimère ADN-ARN) sensiblement complémentaire, constituent des substrats pour des polymérases. Les amorces oligonucléotidiques hybridées sur une séquence d'acide nucléique ont la propriété de fixer à leur extrémité 3'OH une polymérase. La polymérase peut alors allonger l'amorce à partir de son extrémité 3'OH, en présence de nucléotides adéquats, conduisant ainsi à la synthèse d'un brin complémentaire de la séquence matrice sur laquelle est hybridée ladite amorce. Une amorce peut également être constituée par hybridation de l'extrémité d'une séquence d'acide nucléique simple brin sur elle-même, conduisant notamment à la formation de structures secondaires dites en épingle à cheveux ou tiges-boucles.

Par structure "tige-boucle", on désigne toute structure secondaire impliquant au moins une partie nucléotidique, à l'intérieur de laquelle un brin d'une séquence d'acide nucléique s'apparie, par des liaisons hydrogène intramoléculaires, avec une autre partie de la même molécule d'acide nucléique afin de constituer une région "auto-appariée" dite "tige" de nature double brin et une région "boucle" non appariée localisée à une extrémité de ladite tige. Lorsque la boucle est de longueur nulle, on se trouve dans le cas particulier de tige-boucle appelé "épingle à cheveux" ou palindrome. De telles structures "tige-boucle" peuvent notamment se former si une séquence nucléotidique présente deux séquences répétées inversées séparées, par exemple, par au moins un nucléotide ou par un bras de nature hydrocarbonée.

On appelle « agent bloquant » un agent capable de bloquer la réplication d'une matrice d'acide nucléique par une polymérase d'acide nucléique (ADN polymérase, ARN polymérase, transcriptase inverse ou réplicase). De tels agents sont connus. Un agent bloquant peut être un composé nucléique (par exemple un nucléotide modifié), ou non nucléique, qui n'est pas reconnu comme matrice par la polymérase concernée. L'agent bloquant peut être un bras hydrocarboné inséré entre deux nucléotides de la matrice nucléique à l'endroit où l'on désire arrêter la réplication. L'expression « bras hydrocarboné » signifie ici «bras essentiellement hydrocarboné » : il s'agit par exemple d'un bras polyméthylène qui peut être inséré entre deux nucléotides par exemple par réaction d'un diol ou d'une diamine (voir la partie expérimentale ci-après). Un tel groupement polyméthylène peut comporter des substituants ou être interrompu par un ou plusieurs hétéroatomes d'oxygène, de soufre ou d'azote par exemple.

On appelle « réplication » toute réaction catalysée par une polymérase d'acide nucléique, qui aboutit à la copie d'une matrice nucléique, généralement sous la forme de son complémentaire. La polymèrase étant notamment une ADN polymérase, une ARN polymérase, une transcriptase inverse ou une réplicase.

Par "activité de déplacement de brin", on désigne le phénomène par lequel un agent biologique, chimique ou physique, par exemple une ADN polymérase, provoque la dissociation progressive d'un acide nucléique d'avec son brin complémentaire auquel il était apparié, en conjonction avec la néo-synthèse d'un acide nucléique complémentaire dudit brin complémentaire (matrice). Le déplacement de brin commence à l'extrémité 5' de l'acide nucléique ainsi déplacé et se poursuit dans la direction 5' -> 3' tandis que la néo-synthèse d'acide nucléique se poursuit immédiatement en amont du site de déplacement. L'acide nucléique néo-synthétisé (formant un duplex avec la matrice) et l'acide nucléique déplacé obtenu (sous forme de monobrin) ont généralement la même séquence nucléotidique qui est complémentaire du brin d'acide nucléique matrice. L'activité de déplacement de brin peut résider sur la même molécule que celle ayant l'activité de synthèse d'acide nucléique, et particulièrement la synthèse d'ADN, ou elle peut être une activité séparée et indépendante. Des ADN polymérases telles que l'ADN polymérase I d'*E. coli,* le fragment de Klenow de l'ADN polymérase 1, l'ADN polymérase du bactériophage T7 ou T5, la transcriptase inverse du virus HIV sont des enzymes qui possèdent à la fois l'activité polymérase et l'activité de déplacement de brin. Des agents tels que les hélicases peuvent être utilisés en conjonction avec des agents inducteurs qui ne possèdent pas d'activité de déplacement de brin, afin de produire l'effet de déplacement de brin, c'est à dire le déplacement d'un acide nucléique couplé à la synthèse d'un acide nucléique de même séquence. De même, des protéines telles que Rec A ou la Single Strand Binding Protein d'*E. coli* ou d'un autre organisme peuvent être utilisées pour produire ou favoriser le déplacement de brin, en conjonction avec d'autres agents inducteurs. Pour plus de détails et une discussion du déplacement de brin, on peut consulter Kornberg et Baker (1992, DNA Replication, 2nd Édition, p. 113-225, Freeman, New York). La capacité de déplacement de brin permet notamment l'ouverture d'une structure de type tige-boucle, en conjonction avec la synthèse d'au moins une partie de cette structure de type tige-boucle.

Par "promoteur" d'une ARN polymérase, on désigne une séquence ou une structure capable de diriger l'initiation de la transcription. Les séquences promotrices (sous forme de double brin) de différents types d'ARN polymérases sont bien connues. Outre les promoteurs naturels des ARN polymérases, on peut utiliser également des séquences raccourcies dérivées des promoteurs naturels et ayant conservé leur fonctionnalité. D'autres structures capables d'initier la transcription sont par exemple des "bulles" ou "boucles" telles que décrites par Møllegaard *et al.* (1994, *Proc. Natl. Acad. Sci. USA* 91 : 3892-3895) et Aiyar *et al.* (1994. *J. Biol. Chem.* 269 : 13179-13184).

Par séquence "sens" d'un promoteur d'ARN polymérase, on désigne la séquence du brin dudit promoteur (double brin) dont l'extrémité 3' est contiguë au site d'initiation de la transcription qui est défini par ce même promoteur.

Par séquence "anti-sens" d'un promoteur d'ARN polymérase, on désigne la séquence du brin dudit promoteur (double brin) dont l'extrémité 5' est contiguë au nucléotide complémentaire du site d'initiation de la transcription qui est défini par ce même promoteur.

L'invention a donc pour objet un oligonucléotide comprenant, de son extrémité 5' vers son extrémité 3' :
- une première partie capable d'auto-appariement pour former une structure tige-boucle comprenant un segment amont et un segment aval appariés,
- et une seconde partie non susceptible d'auto-appariement,
caractérisé par le fait que ladite première partie comprend au moins un agent bloquant, capable de bloquer la réplication par une polymérase dudit oligonucléotide, de façon qu'au moins une partie du segment aval soit répliquée et de façon que le segment amont ne soit répliqué ni totalement ni partiellement.

L'agent bloquant est par exemple tel que la partie susceptible d'être répliquée du segment aval a une longueur d'au moins 2 nucléotides, et en particulier d'au moins 3 nucléotides, ou d'au moins 5 nucléotides.

L'extrémité aval du segment amont et l'extrémité amont du segment aval de l'oligonucléotide peuvent être reliées par une boucle nucléique ou non nucléique.

Les segments amont et aval peuvent contenir chacun de 2 à 30, et en particulier de 3 à 15 nucléotides.

L'agent bloquant comprend par exemple au moins un nucléotide modifié non recopiable par une polymérase, ou un bras hydrocarboné. La boucle peut contenir l'agent bloquant.

L'oligonucléotide peut contenir, entre les première et deuxième parties, une troisième partie contenant la séquence sens d'un promoteur pour une ARN polymérase.

Selon un mode de réalisation particulier, l'oligonucléotide de l'invention est caractérisé par le fait que ladite deuxième partie contient de 5 à 40 nucléotides.

L'oligonucléotide de l'invention peut être utilisé comme amorce dans un procédé d'amplification d'une séquence cible d'un acide nucléique, ladite séquence cible comprenant une séquence aval d'au moins 5 nucléotides, et dans ce cas ladite deuxième partie contient à son extrémité 3' une séquence capable de s'hybrider avec ladite séquence aval de la cible.

L'invention concerne également un procédé d'amplification cyclique d'une séquence cible d'un acide nucléique, ladite séquence cible comprenant à son extrémité 5' une région amont et à son extrémité 3' une région aval, comprenant les étapes consistant à :
- obtenir un polynucléotide simple brin comprenant un premier segment correspondant à la séquence cible à amplifier, un second segment situé en amont de l'extrémité 5' du premier segment et un troisième segment situé en aval de l'extrémité 3' du premier segment,
   ledit second segment dudit polynucléotide simple brin étant homologue de ladite première partie d'un oligonucléotide tel que défini ci-dessus,
   et ledit troisième segment dudit polynucléotide simple brin ayant une séquence quelconque,
- mettre ledit polynucléotide en présence de :
   (a) une première amorce telle que définie ci-dessus, capable de s'hybrider avec la région aval de la séquence cible, étant entendu qu'au moins la partie aval du segment aval de la première partie de la première amorce est complémentaire du troisième segment du polynucléotide simple brin,
      éventuellement une seconde amorce telle que définie ci-dessus, capable de s'hybrider avec une région aval de la séquence complémentaire de la séquence cible,
      cette région aval étant complémentaire de ladite région amont de la séquence cible,
      étant entendu que la première partie de la seconde amorce est celle dont le second segment dudit polynucléotide simple brin est l'homologue,
   (b) une troisième amorce dont la séquence est homologue d'au moins une partie aval de la séquence du segment aval de ladite première partie de la première amorce,
   (c) une quatrième amorce dont la séquence est homologue d'au moins une partie de la séquence du segment aval de ladite première partie constituant ledit second segment dudit polynucléotide simple brin,
   et (d) un système enzymatique contenant au moins une activité de réplication dudit acide nucléique et une activité de déplacement de brin,
- et faire incuber le mélange obtenu dans des conditions permettant l'hybridation et le fonctionnement desdites activités enzymatiques.

Dans un mode de réalisation particulier de ce procédé, la première amorce peut contenir la séquence sens d'un promoteur, comme mentionné plus haut, et le procédé est alors caractérisé par le fait que ledit polynucléotide simple brin contient, entre son premier segment et son troisième segment, un quatrième segment complémentaire de ladite séquence sens contenue dans la première amorce, et par le fait que ledit système enzymatique contient en outre une activité d'ARN polymérase sous la dépendance dudit promoteur.

Selon un autre mode de réalisation particulier non exclusif du mode de réalisation particulier précédent, la seconde amorce est présente et contient également une séquence sens d'un promoteur, et le procédé est alors caractérisé par le fait que ledit polynucléotide simple brin contient, entre son premier segment et son deuxième segment, un segment supplémentaire dont la séquence est celle de la séquence sens contenue dans la deuxième amorce, et que ledit système contient en outre une activité d'ARN polymérase sous la dépendance dudit promoteur correspondant à cette séquence sens contenue dans la deuxième amorce.

Des modes de réalisation particuliers vont maintenant être décrits en faisant, le cas échéant, référence aux dessins annexés dans lesquels :

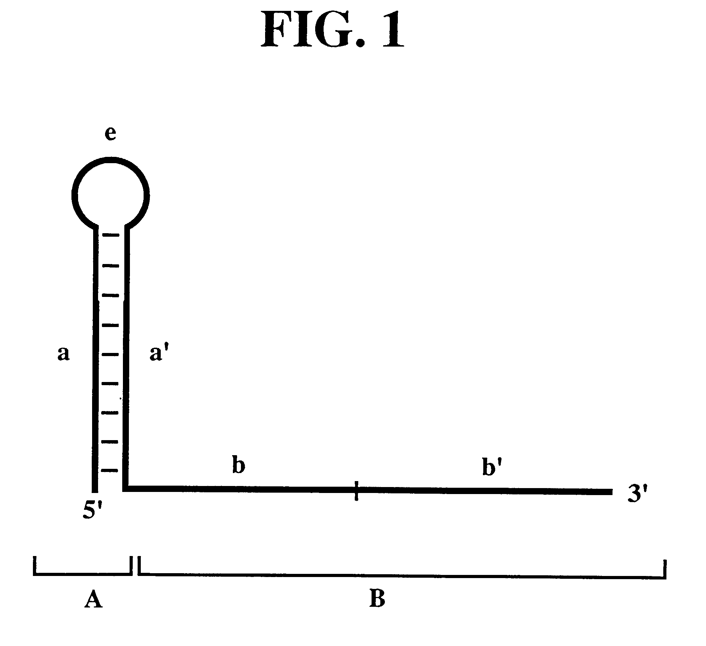

La figure 1 décrit un oligonucléotide selon l'invention composée d'une séquence capable de former une structure de type auto-appariée tige-boucle et renfermant au moins un agent bloquant l'élongation par une polymérase d'acide nucléique. Les lignes verticales, segments (*a*) et (*a*'), représentent une séquence d'acide nucléique appartenant à la tige (région auto-appariée) et la partie en arc de cercle une structure de nature nucléique et/ou hydrocarbonée formant la boucle, segment (*e*). Les lignes horizontales fines discontinues symbolisent les liaisons hydrogène établies entre les nucléotides complémentaires hybridés au sein de la tige. La polarité des acides nucléique est indiquée par leurs extrémités 5' et 3'.

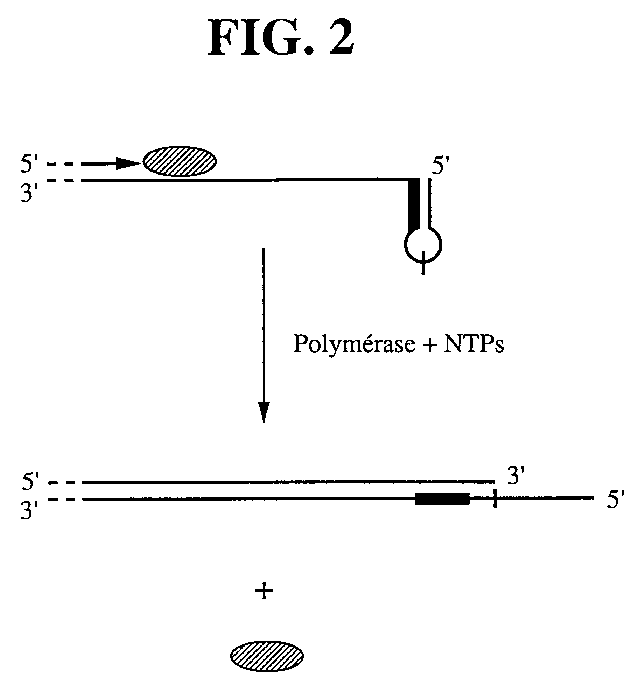

La figure 2 décrit la réplication d'une molécule comportant une structure de type tige-boucle et renfermant au moins un agent bloquant l'élongation par une polymérase d'acide nucléique, à l'aide par exemple d'une ADN polymérase ou une ARN polymérase (ADN et/ou ARN-dépendantes) en présence de désoxyribonucléosides triphosphates (dNTPs) ou de ribonucléosides triphosphates (rNTPs), respectivement. La réplication s'opère par exemple par élongation de 5' vers 3' d'une amorce oligonucléotidique, hybridée du côté de l'extrémité 3' d'un acide nucléique matrice contenant une structure de type tige-boucle. Les lignes droites représentent une séquence d'acide nucléique et la partie en arc de cercle une structure formant la boucle. Le trait transversal situé dans la région de la boucle désigne l'endroit à partir duquel la réplication de la tige boucle se trouve bloquée. Il s'ensuit un décrochement de la polymérase (symbolisée par l'ellipse hachurée) de sa matrice. Le segment fortement épaissi indique la région de la tige recopiée par la polymérase. Les traits pointillés représentent les extrémités non définies des acides nucléiques.

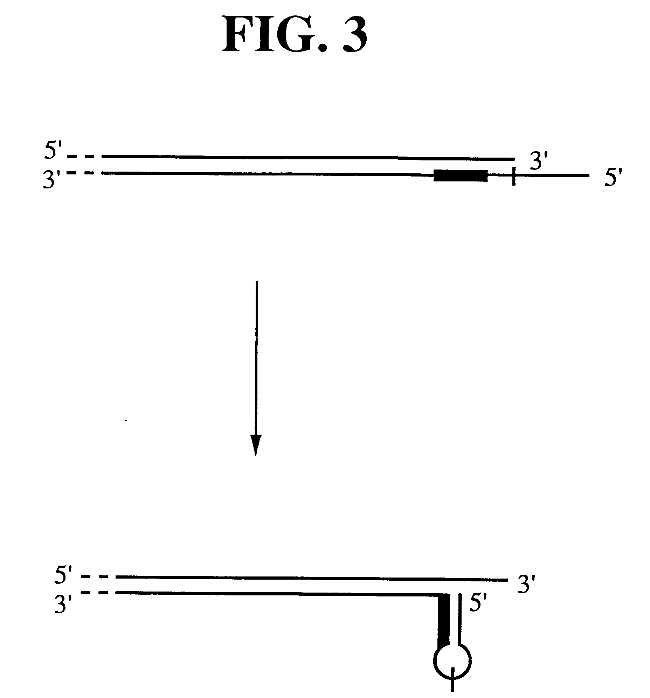

La figure 3 décrit comment en déplaçant l'équilibre thermodynamique on peut obtenir la partie A des oligonucléotides sous la forme d'une structure moléculaire auto-appariée tige-boucle à partir d'une molécule duplex. Les lignes droites représentent une séquence d'acide nucléique et la partie en arc de cercle une structure formant une boucle. Le trait court transversal tracé dans la région de la boucle désigne l'endroit au-delà duquel la réplication de la tige boucle ne peut plus se produire. Le trait fortement épaissi indique la région de la tige recopiée par la polymérase. Les traits pointillés représentent les extrémités non définies des acides nucléiques.

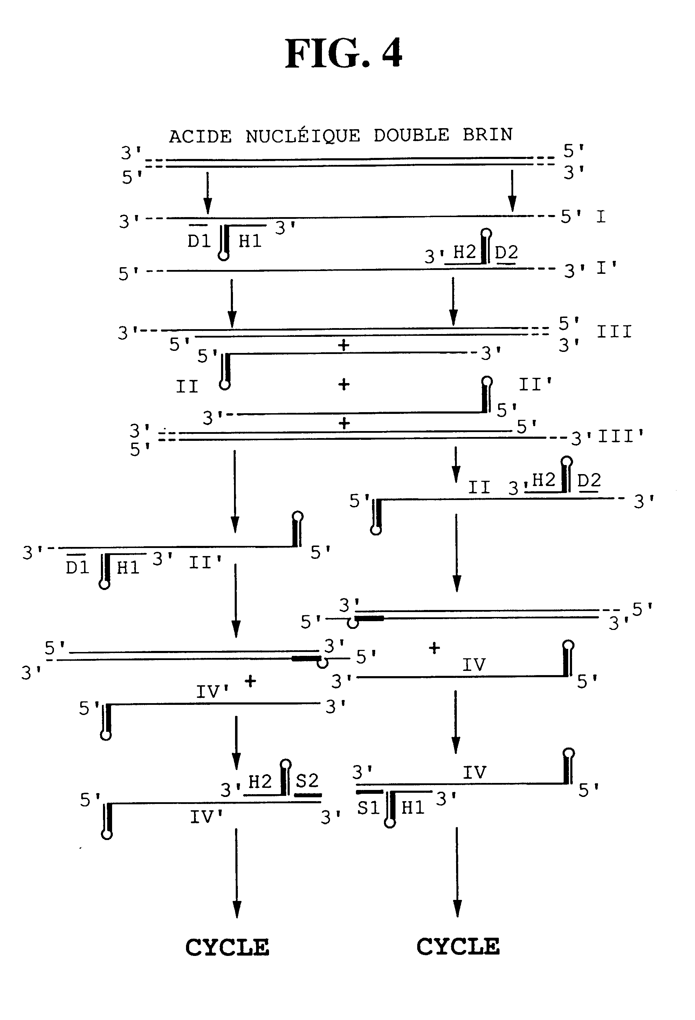

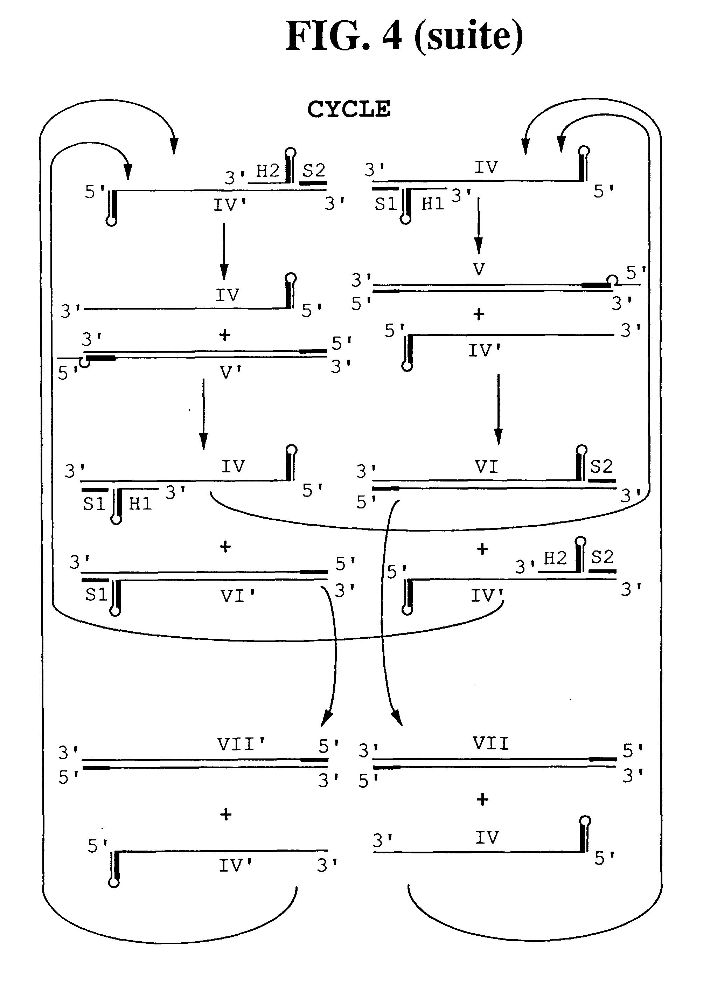

La figure 4 représente schématiquement un cas particulier d'amplification, conformément à l'invention, d'acides nucléiques utilisant un oligonucléotide comprenant une structure tige-boucle. Les lignes droites représentent une séquence d'acide nucléique et la partie en arc de cercle une structure formant la boucle. Le segment fortement épaissi indique la région de la tige recopiée par la polymérase, comme indiqué à propos de la figure 3.

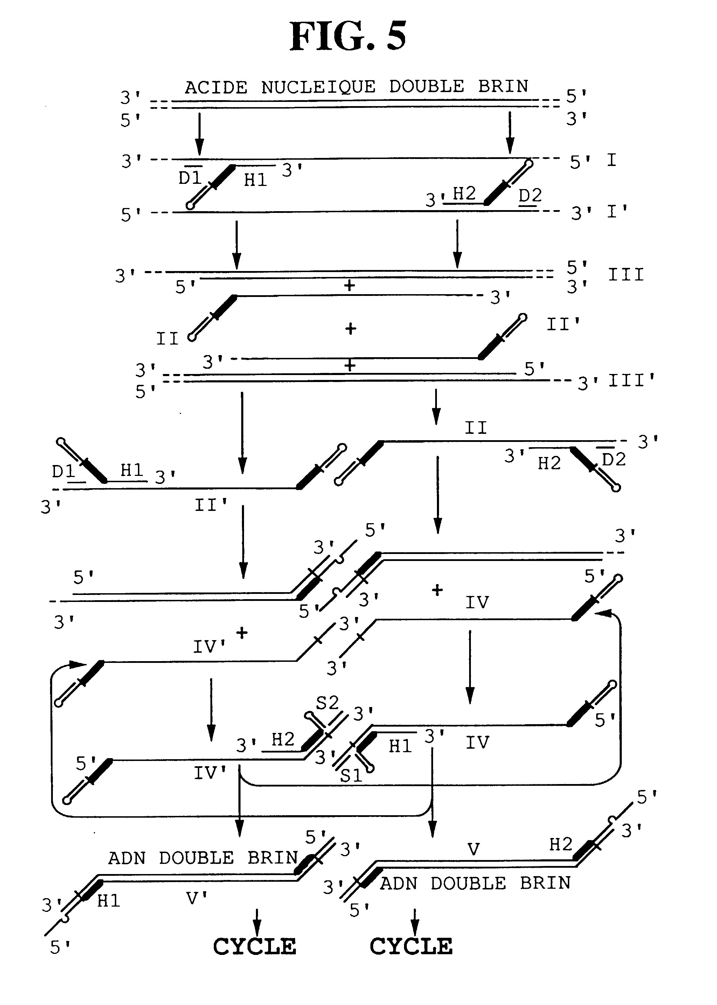

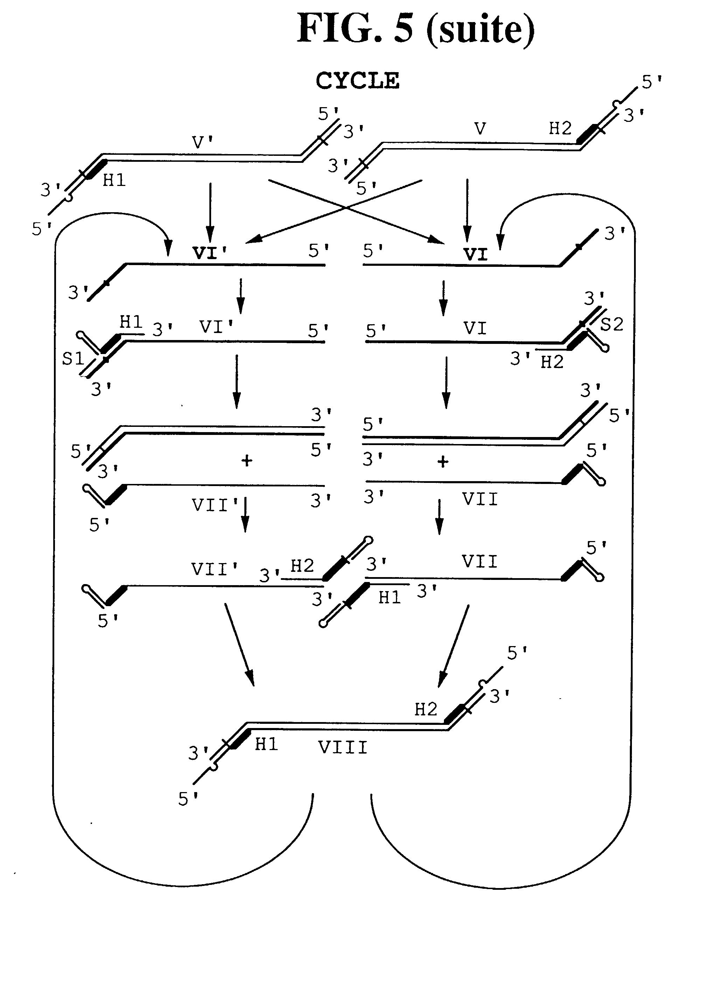

La figure 5 représente schématiquement un autre cas particulier d'amplification, conformément à l'invention, d'acides nucléiques utilisant un oligonucléotide comprenant une structure tige-boucle et renfermant en outre une séquence sens d'un promoteur pour une ARN polymérase. Les lignes droites en traits fins représentent une séquence d'ADN, les traits épais représentant de l'ARN. Le segment fortement épaissi indique une région contenant une séquence sens de promoteur d'ARN polymérase. La partie en arc de cercle représente une structure formant la boucle.

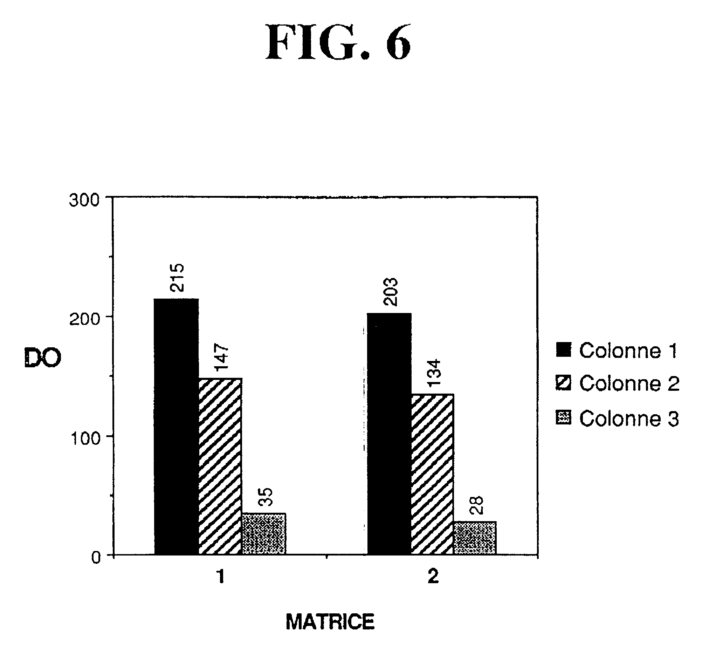

La figure 6 représente un histogramme des résultats obtenus conformément à l'exemple 2. La densité optique (DO) indiquée en ordonnée est chiffrée en millièmes. Le type de matrice est indiqué en abscisse : ARN (1) ou ADN (2). La colonne (noire) représente les essais en présence de tous les réactifs, y compris la transcriptase inverse et l'amorce de déplacement. La colonne 2 (hachurée) représente les essais en présence de tous les réactifs à l'exception de l'amorce de déplacement DIS5. La colonne 3 (grisée) représente les essais en présence de tous les réactifs à l'exception de la transcripase inverse.

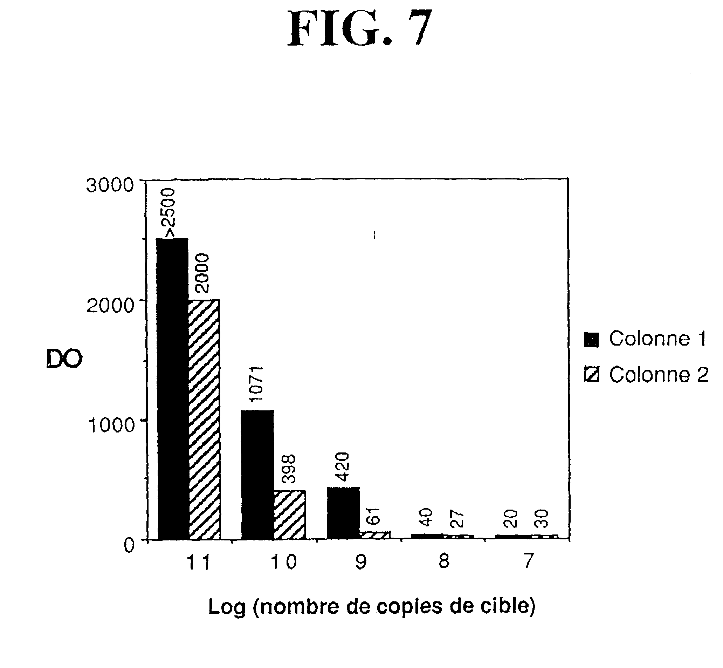

La figure 7 représente un histogramme des résultats obtenus conformément à l'exemple 5. La densité optique (DO) indiquée en ordonnée est chiffrée en millièmes. Le logarithme du nombre de copies de cible par essai est indiqué en abscisse. La colonne 1 (noire) représente les essais en présence de tous les réactifs, y compris la transcriptase inverse et l'amorce de déplacement. La colonne 2 (hachurée) représente les essais en présence de tous les réactifs à l'exception des amorces de déplacement E220 et DIS8.

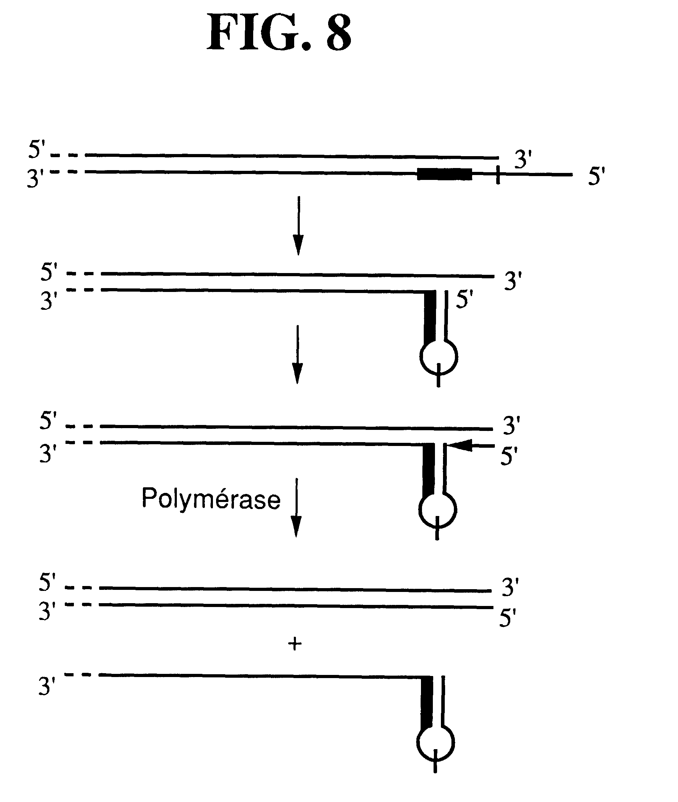

La figure 8 représente schématiquement le principe de l'étude du déplacement de brins contenant des structures tige-boucle au sein d'un duplex ADN, à l'aide d'une ADN polymérase, tel que décrit dans l'exemple 9. Les traits fortement épaissis indiquent la région de la tige de la structure tige-boucle recopiée par la polymérase.

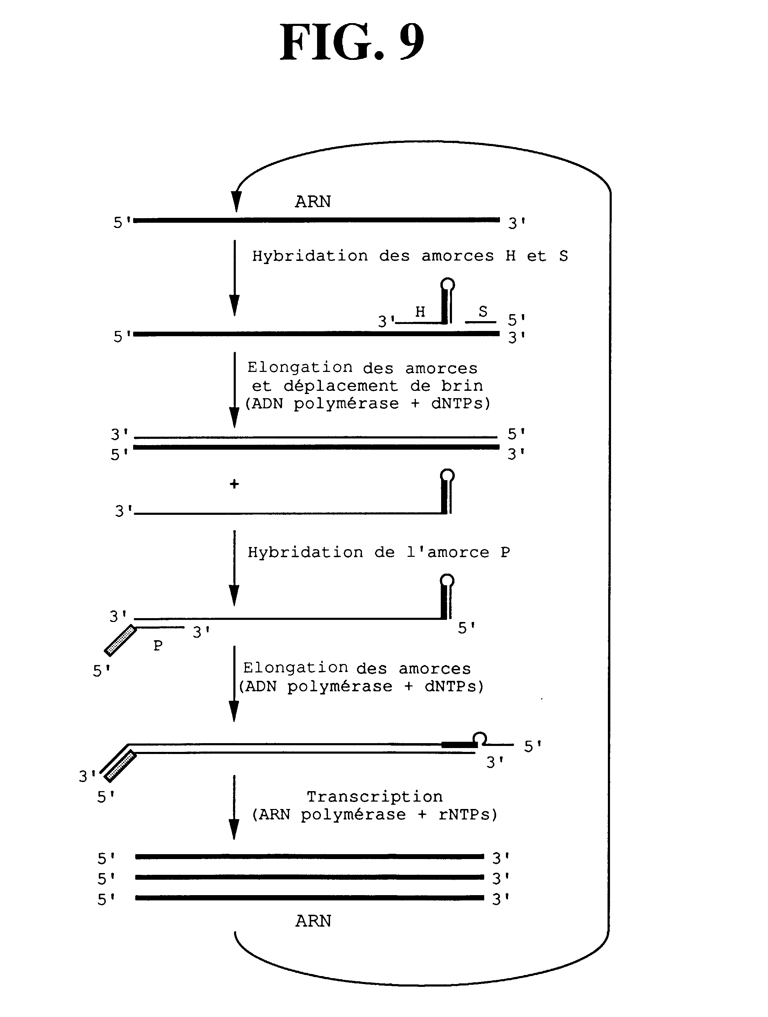

La figure 9 représente schématiquement un autre cas particulier d'amplification, conformément à l'invention, d'acides nucléiques utilisant un oligonucléotide comprenant une structure tige-boucle et renfermant en outre une séquence sens d'un promoteur pour une ARN polymérase. Les lignes droites en traits fins représentent une séquence d'ADN, les traits épais représentant de l'ARN. La partie en arc de cercle représente une structure formant la boucle.

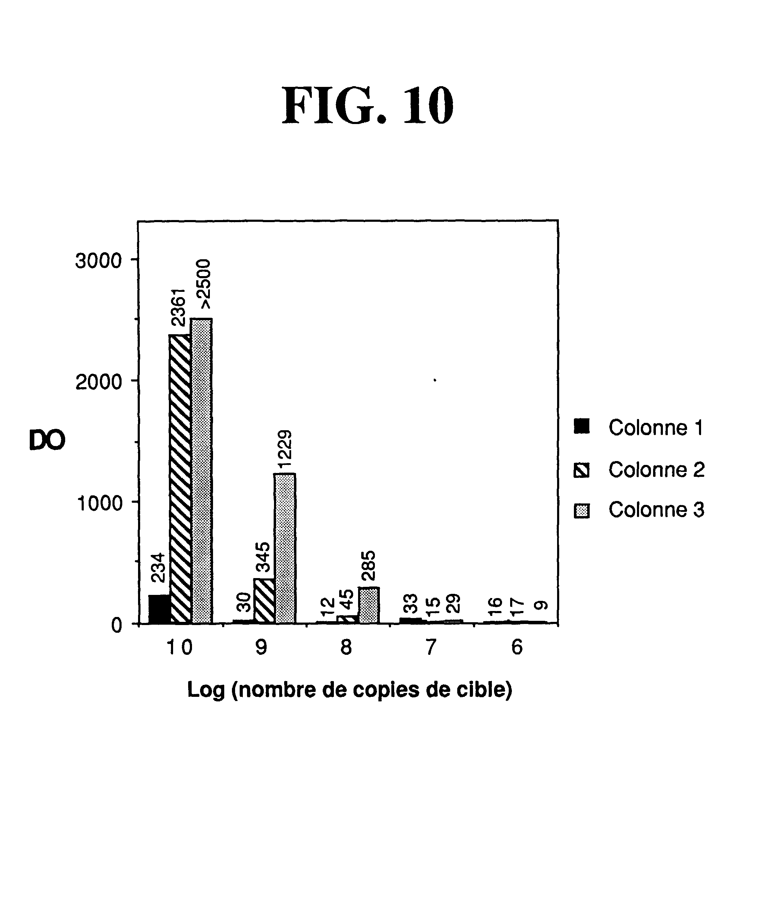

La figure 10 représente un histogramme des résultats obtenus conformément à l'exemple 10. La densité optique (DO) indiquée en ordonnée est chiffrée en millièmes. Le logarithme du nombre de copies de cible par essai est indiqué en abscisse. La colonne 1 (noire) représente les essais en présence de tous les réactifs à l'exception des enzymes. La colonne 2 (hachurée) représente les essais en présence de tous les réactifs à l'exception de l'amorce. de déplacement Dis 22. La colonne 3 (grisée) représente les essais en présence de tous les réactifs, y compris l'amorce tige-boucle.

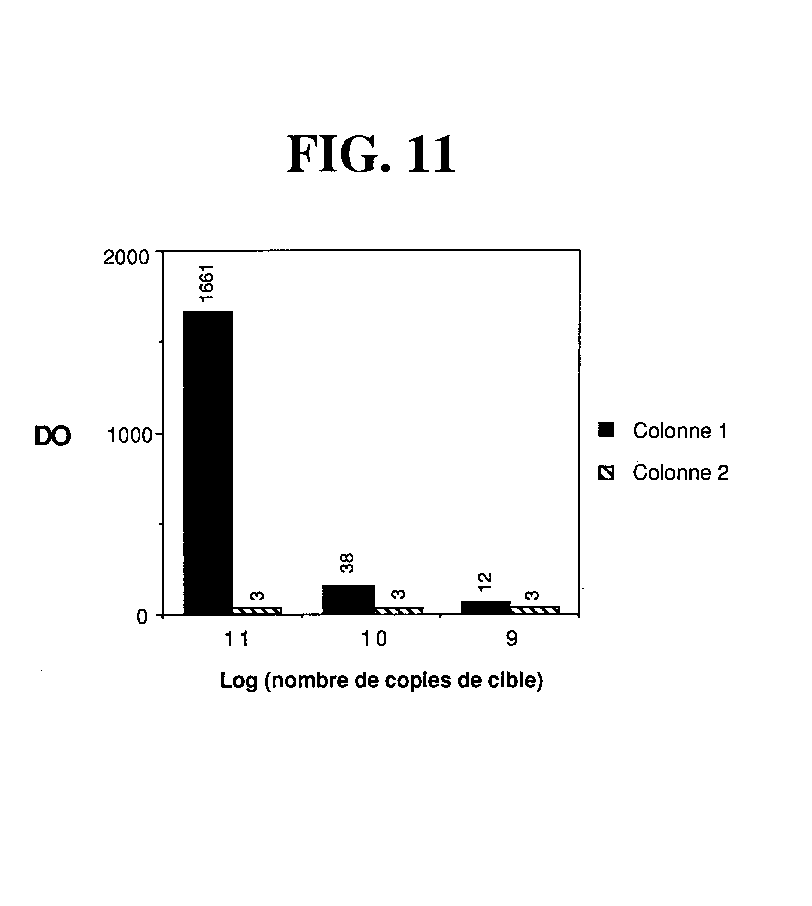

La figure 11 représente un histogramme des résultats obtenus conformément à l'exemple 11. La densité optique (DO) indiquée en ordonnée est chiffrée en millièmes. Le logarithme du nombre de copies de cible par essai est indiqué en abscisse. La colonne 1 (noire) représente les essais en présence de tous les réactifs. La colonne 2 (hachurée) représente les essais en présence de tous les réactifs à l'exception de l'enzyme.

Le procédé qui va être décrit ci-dessous peut fonctionner à température constante et notamment à la température optimale de l'ADN polymérase utilisée. Par ailleurs, les amorces selon l'invention utilisées pour la réalisation de ce procédé seront désignées dans la suite par la lettre H assortie éventuellement d'une numérotation si plusieurs de ces amorces entrent en jeu.

La présente invention a notamment pour objet un oligonucléotide, tel que présenté dans la figure 1, comprenant successivement de son extrémité 5' vers son extrémité 3' :
- une première partie A, au moins partiellement nucléique, comprenant de 5' en 3' deux segments nucléiques (*a*) et (*a*'), auto-complémentaires, capables d'auto-appariement pour former une tige, séparés par un troisième segment facultatif et non obligatoirement nucléique *(e)* qui lorsque ladite tige est formée peut constituer notamment une boucle non appariée, l'ensemble de ladite partie A constituant alors une structure moléculaire auto-appariée dite tige-boucle, et ladite partie A comprenant au moins un agent bloquant l'élongation par une polymérase d'acide nucléique,
- une seconde partie B, de nature nucléique, comprenant de 5' en 3', un premier segment facultatif *(b)* et un second segment *(b),* dont au moins ledit segment *(b)* est capable d'hybridation avec une séquence nucléique cible.

Dans des conditions expérimentales déterminées, les segments (*a*) et (*a*') s'associent pour former une tige, alors que, s'il est présent, le segment *(e)* peut constituer notamment une boucle non appariée ; cet ensemble est alors appelé tige-boucle. Bien entendu, il est à la portée de l'homme du métier d'adapter la composition du segment *(e)* afin que ledit segment, lorsque les segments (*a*) et (*a*') sont auto-appariés, adopte une structure quelconque, autre qu'une boucle, qu'elle que soit la complexité, le nombre de boucles et/ou d'appariements mis en jeu dans la formation de ladite structure.

Selon la présente invention, cette partie A de l'oligonucléotide comprend au moins un agent bloquant l'élongation par une polymérase d'acide nucléique, c'est à dire un agent capable de bloquer la réplication ou la transcription qui ne devra obligatoirement pas s'étendre au delà de l'extrémité 3' de la région définie par le segment (*a*) dudit oligonucléotide (Figure 2) et de manière préférentielle seule la séquence (*a*') sera recopiable. Par ailleurs, ladite partie A, impliquée dans un duplex avec son brin complémentaire néo-synthétisé, est capable de former une structure moléculaire au moins partiellement auto-appariée à la suite du blocage de la réplication ou la transcription (Figure 3).

Les segments (*a*) et *(a')* sont des séquences nucléiques, ADN et/ou ARN, présentant une auto-complémentarité, c'est à dire qu'il s'agit de deux séquences répétées inversées (par rapport à un même axe 5'-3' ou 3'-5'). Néanmoins, les séquences de (*a*) et de (*a*') peuvent présenter des nucléotides non complémentaires dans la mesure où ceux-ci n'empêchent pas la formation de ladite tige. Par ailleurs, chacune de ces séquences (*a*) et (*a*') peut comporter des nucléotides modifiés ou des composants non nucléiques à la condition que ces modifications n'empêchent pas la formation de ladite tige.

Le segment *(e)* est facultatif et comporte soit une séquence nucléique, soit une structure non nucléique. La composition de ce segment *(e)* ne doit pas inhiber la formation de ladite structure auto-appariée tige-boucle selon la présente invention. Si ce segment comporte au moins une séquence nucléotidique, celle-ci sera choisie de manière à ne présenter aucune complémentarité avec elle-même, ou avec les segments (*a*) et (*a*').

En fait, le choix des composants de ces segments *(a), (a') et (e)* est dicté par la nécessité d'avoir au moins un agent bloquant l'élongation par une polymérase d'acide nucléique, situé dans une région localisée à partir du troisième nucléotide défini à partir de l'extrémité 3' du segment (*a*') jusqu'au nucléotide de l'extrémité 3' du segment *(a)* dudit oligonucléotide et préférentiellement en une position quelconque du segment *(e),* si celui-ci est présent. Dans le cas où le segment (*e*) est absent de l'oligonucléotide, seul le segment (*a*') ou au moins une partie de (*a*') doit être recopiable par une polymérase d'acide nucléique. Dans ce cas, ledit élément bloquant la polymérisation pourra être localisé par exemple à l'extrémité 3' du segment (*a*).

Cet arrêt de la réplication ou de la transcription par une polymérase peut être obtenu par la présence de bases modifiées non recopiables par une polymérase, de structures dont les sucres sont modifiés, ou encore de structures pouvant s'hybrider sur des acides nucléiques mais dont la nature est différente, telles que les PNA (Nielsen *et al.,* 1991. *Sience* 254 : 1497-1500; ⌀rum *et al.,* 1993. *Nucleic acids Res.* 21 : 5332-5336). Ce blocage peut également être obtenu par l'insersion de bras de nature hydrocarbonée tels qu'un bras hexaéthylène glycol (Zhang et al., 1991, *Nucleic acid Res,* 19, 3929-3936) ou de nature amine aliphatique primaire (Kessler, 1992, Nonisotopic DNA Probe Techniques, ed. Kricka, academic Press Inc., New York, pp 58). D'autres bras utilisables sont décrits par Uhlmann et Peyman (1990, Chemical Reviews, 90, 544-584). Des sites abasiques peuvent également être utilisés pour le blocage de l'élongation (Zhou et Doetsch, 1993, *Proc. Natl. acad. Sci.* USa 90, 6601-6605). On peut aussi introduire des lésions telles que des dimères cyclobutane-pyrimidine induits par irradiation aux rayonnements ultra-violet qui sont connus comme agents bloquants des ARN polymérases.

L'obtention des oligonucléotides selon l'invention sous une forme auto-appariée est rendue possible par des conditions permettant d'obtenir un déplacement de l'équilibre thermodynamique d'une structure non auto-appariée vers une structure auto-appariée tige-boucle qui, si la boucle est de taille réduite à zéro nucléotide, constituera une structure en épingle à cheveux. Ces conditions sont connues ou peuvent être déterminées dans chaque cas particulier par des expériences de routine, par exemple en faisant varier les conditions de tampon et de température. Ces propriétés d'équilibre ont été décrites dans la littérature pour quelques séquences oligonucléotidiques particulières (Hirao *et al.,* 1994. *Nucleic acids Res.* 22 : 576-582; Yoshizawa *et al.,* 1994. *Nucleic acids Res.* 22 : 2217-2221; Durand *et al.,* 1990. *Nucleic acids Res.* 18 : 6353-6359). Ainsi, il a été montré que des oligodésoxynucléotides même très courts, tels que par exemple d(GCGAAGC), sont capables de former des structures en épingle à cheveux extrêmement stables, résistantes à la chaleur et aux nucléases. Par ailleurs, l'influence de la séquence sur la transition de la forme non auto-appariée à la forme auto-appariée tige-boucle d'oligonucléotides a été étudiée (Xodo *et al.,* 1989. *Biochimie* 71 : 793-803). Ces auteurs ont ainsi montré que le repliement des oligonucléotides n'est pas induit par la présence de nucléotides non appariés situés entre les séquences répétées inversées car des oligonucléotides constitués de la séquence (^{5'}CG^{3'})ₙ possèdent une forte propension à former une structure intramoléculaire. De plus, il s'avère que les séquences qui sont le moins enclines à la formation de structures tige-boucle sont celles dont la tige est formée d'une série d'appariemments A-T et d'appariemments G-C aux extrémités de la boucle.

La composition en base de la tige intervient de manière importante dans la stabilité thermique de la tige; en particulier, la substitution d'une paire C-G par une paire A-T, au niveau de la jonction de la tige et de la boucle, abaisse la stabilité de la structure de 9°C. D'autres auteurs ont montré l'importance de la boucle sur la stabilité des structures tige-boucle. Ainsi, dans le cas d'une boucle de 4 nucléotides, cette stabilité est diminuée si la paire de base ^{5'}C-G^{3'} fermant la boucle est remplacée par ^{5'}G-C^{3'}, qu'il s'agisse de molécules d'ADN ou d' ARN (Antao et Tinoco, 1992. *Nucleic acids Res.* 20 : 819-824). Les boucles de type ^{5'}GNRA^{3'} sont celles dont la séquence assure la meilleure stabilité des structures tige-boucle ARN. L'influence de boucles de nature non nucléotidique sur la stabilité des structures tige boucle a également été étudiée. En particulier, des structures de type oligodésoxyribonucléotide formant une tige-boucle dont la boucle est une chaîne provenant d'un couplage avec l'hexaéthylèneglycol ont été décrites (Durand *et al.,* 1990. *Nucleic acids Res.* 18 : 6353-6359).

La longueur de la boucle est aussi un facteur important pour la stabilité de la structure tige-boucle. Rentzeperis *et al.* (1993. *Nucleic acids Res.* 21 : 2683-2689) ont fait varier la longueur d'une boucle poly-T de 3, 5 ou 7 nucléotides et montrent ainsi que pour une tige donnée la stabilité des structures étudiées décroit avec l'augmentation de la taille de la boucle. Groebe et Uhlenbeck (1988, *Nucleic acids Res.* 16 : 11725-11731) ont par ailleurs montré qu'une boucle de 4 à 5 nucléotides confère le maximum de stabilité à une structure tige-boucle de nature ARN.

En outre, il a été montré que la forme auto-appariée tige-boucle est privilégiée à faible force ionique et en présence de MgCl₂ (Boulard *et al.,* 1991. *Nucleic acids Res.* 19 : 5159-5167).

En ce qui concerne la taille des segments (*a*), (*a*') et *(e)* des oligonucléotides, on choisira par exemple des tailles variant de 2 à 30 nucléotides, et notamment de 3 à 15 nucléotides pour le segment *(a)*, de 2 à 30 nucléotides, et notamment de 3 à 15 nucléotides pour le segment (*a*') et de 0 à 20 nucléotides, et notamment de 2 à 8 nucléotides pour le segment *(e).*

Les oligonucléotides décrits dans la présente demande renferment une partie B (figure 1) localisée immédiatement à l'extrémité 3' du segment (*a*') de la partie A desdits oligonucléotides. Cette partie B renferme successivement, de 3' en 5', au moins un segment (*b*') éventuellement associé à un segment (*b*)*.*

Les segments (*b*) et (*b*') sont des séquences nucléiques ADN et / ou ARN. Le segment (*b*) est facultatif et lorsqu'il est présent, il peut notamment renfermer un promoteur pour une ARN polymérase, un site de reconnaissance pour une enzyme de restriction, une enzyme reconnaissant une origine de réplication. Les séquences promotrices, ou encore appelées séquences sens ou anti-sens de promoteur, peuvent notamment être des séquences promotrices d' ARN polymérases phagiques (par exemple des bactériophages T7, T3 ou SP6 représentées par les séquences SEQ ID N°19, SEQ ID N°16 et SEQ ID N°20, respectivement). Par ailleurs, il est connu que l'ARN polymérase du phage T7 requiert la présence d'un promoteur spécifique sur l'ADN pour une transcription efficace d'ARN. La séquence de ce promoteur spécifique est parfaitement caractérisée (Dunn et Studier, 1983. *J. Mol. Biol.* 166 : 477-535) et la grande spécificité de transcription de l'ARN polymérase T7 à partir de son promoteur a été mise en évidence (Bailey *et al.,* 1983. *Proc. Natl. acad Sci.* 80 : 2814-2818). Ces propriétés peuvent être utilisées afin de produire *in vitro* des ARNs à l'aide d'ARN polymérase T7 à partir de matrices contenant un promoteur double brin fonctionnel (Krupp et Söll, 1987. *FEBS Lett.* 2 : 271-275 ; Milligan *et al.,* 1987. *Nucl. acids Res.* 15 : 8783-8798). L'article de Krupp (1988. *Gene* 72 : 75-89) constitue une bonne revue des modes d'utilisation des ARN polymérases phagiques et des stratégies utiles pour la synthèse d'ARN *in vitro.* De plus, cette séquence promotrice lorsqu'elle est présente peut être assortie d'une séquence, dite séquence d'initiation, renfermant la séquence naturellement présente à proximité du nucléotide +1 sur lequel se produit l'initiation de la transcription, et localisée en aval du promoteur T7, T3 ou SP6 sur le segment (*b*)*.* Ces séquences sont désignées par SEQ ID N°29 pour la T7, SEQ ID N°30 pour la T3 et SEQ ID N°31 pour la SP6.

Le segment (*b*') pour sa part est constitué par toute séquence capable de s'hybrider spécifiquement et de manière stable à une séquence donnée d'un acide nucléique cible. Bien que la longueur de cette séquence ne soit pas un facteur capital concernant notamment la stabilité ou les propriétés des oligonucléotides décrits précédemment, le segment (*b*') pourra avoir une longueur de 5 à 40 nucléotides, et notamment de 10 à 20 nucléotides, afin d'obtenir une hybridation rapide, spécifique et stable de ce segment (*b*') avec une séquence cible.

La présente invention a également pour objet un oligonucléotide selon l'invention utilisable comme amorce notamment dans un procédé d'amplification d'une séquence cible d'un acide nucléique comprenant à son extrémité 3' une séquence aval d'au moins 5 nucléotides, caractérisé en ce qu'au moins le segment (*b*') dudit oligonucléotide est capable de s'hybrider avec ladite séquence aval.

Les amorces selon la présente invention permettent notamment de développer un procédé d'amplification cyclique d'une séquence cible d'un acide nucléique et/ou d'une séquence complémentaire de ladite séquence cible, comme indiqué ci-dessus.

Ce procédé d'amplification présente les avantages d'être isotherme, de pouvoir n'utiliser qu'une seule enzyme, d'être réalisable aussi bien à partir de cible ARN que de cible ADN, avec ou sans extrémité définie, de ne pas être limité par la séquence de la cible et de ne pas nécessiter d'activité nucléase pour la séparation des brins d'un duplex ou d'incorporation de nucléotides modifiés dans les produits d'amplification, par la polymérase utilisée.

Cette méthode, appelée "RDS" ou "Réaction de Déplacement de Structures secondaires", requiert par exemple l'utilisation d'une ADN polymérase (ARN ou ADN dépendante) associée à une activité de déplacement de brin, cette activité pouvant être apportée par la polymérase elle-même (Masamune et Richardson, 1971. *J. Biol. Chem.* 246 : 2992-2701; Lundquist et Olivera, 1992.*Cell* 31 : 53-60).

La polymérase utilisée dans le procédé de l'invention est préférentiellement pourvue d'une activité de déplacement de brin. Cette activité est une propriété bien connue de certaines ADN polymérases (Sambrook *et al.,* 1989. Molecular Cloning : a Laboratory Manual, 2nd Edition, pp. 5.33-5.35, Cold Spring Harbor Laboratory, Cold Spring Harbor). Les propriétés des ADN polymérases, et notamment l'activité de déplacement de brin de certaines d'entre elles sont détaillées par Kornberg et Baker (1992. DNA Replication, 2nd Edition, pp.113-225, Freeman, New York). L'activité de déplacement de brin a été mise en évidence initialement pour le fragment de Klenow de l'ADN polymérase I *d'Escherichia coli* (Masamune et Richardson, 1971. *J. Biol. Chem.* 246 : 2692-2701) qui permet d'initier la réplication d'un acide nucléique à partir de l'extrémité 3'OH d'une césure sur un ADN double brin. Cette propriété de déplacement de brin est limitée dans le cas où les ADN polymérases possèdent une activité 5'-3' exonucléase (Lundquist et Olivera, 1982. *Cell* 31 : 53-60). Cette activité de déplacement de brin a également été mise en évidence chez des ADN polymérases thermostables telles que la *Tli* ADN polymérase (Kong *et al.,* 1993. *J. Biol. Chem.* 268 : 1965-1975). Dans ce cas, il a également été montré que des formes mutées de cette enzyme, ne présentant pas d'activité 5'-3' exonucléase, possèdent une plus grande capacité de déplacement de brin. Le déplacement de brin n'est pas une propriété commune à toutes les ADN polymérases, puisque certaines d'entre elles, comme la T4 ADN polymérases, ne sont pas capables de réaliser, seules, le déplacement de brin. Cette activité de déplacement de brin a également été mise en évidence pour la T7 ADN polymérase (Lechner *et al.,* 1983. *J. Biol. Chem.* 258 : 11174-11184) et pour la transcriptase inverse d'HIV (Huber *et al.,* 1989. *J. Biol. Chem.* 264 : 4669-4678). De manière préférentielle, une ADN polymérase dépourvue d'activité 5'-3' exonucléase est utilisée pour la réalisation du cycle d'amplification selon la présente invention. Le fragment de Klenow de l'ADN polymérase I d'*Escherichia coli* constitue un exemple de polymérase dépourvue d'activité 5'-3' exonucléase, de même que des polymérases telles que la T4 ADN polymérase, la T7 ADN polymérase ou la Sequenase™ (US Biochemical), la T5 ADN polymérase ou la Phi29 ADN polymérase pourraient également être utilisées. Des enzymes telles que la *Vent* (exo-)™ (New England Biolabs), le fragment de Klenow de l'ADN polymérase I d'*Escherichia coli* (Boehringer Manheim), le fragment de Stoffel de la *Taq* polymerase (applied Biosystems) ou la *Bca* ADN polymerase (Takara Shuzo) peuvent également être utilisées. Ces enzymes ont en particulier la capacité de réaliser la polymérisation des acides nucléiques à partir de matrices possédant des structures secondaires importantes, telles que les structures tige-boucle. Plus particulièrement, il a été montré que le grand fragment de l'ADN polymérase I de *Bacillus stearothermophilus* possède une grande capacité à résoudre les structures tige-boucle (Ye et Hong, 1987. *Sci. Sin.* 30 : 503-506). Le choix de la polymérase utilisée pourra également varier en fonction de la température à laquelle on souhaite travailler et en fonction du type d'acide nucléique de départ (ARN ou ADN). En particulier, on peut utiliser la Transcriptase Inverse MMLV Superscript^{II}™, dépourvue d'activité RNase H (Gibco-BRL), dont la processivité est grande et dont nous avons pu vérifier la capacité de déplacement de brin, pour réaliser une amplification d'ADN à partir d'une cible initiale ARN. Toute enzyme possédant une activité ADN polymérase ARN- et/ou ADN-dépendante peut donc être utilisée dans la présente invention si elle possède une activité de déplacement de brin. Dans le cas contraire, l'activité de déplacement de brin peut être conférée par un agent inducteur, une activité de type hélicase ou RecA. Les propriétés de RecA, notamment dans le processus de réassociation d'ADN simple brin, de capture de brin ou d'assimilation de brin sont détaillées par McEntee et Weinstock (1981. *The Enzymes,* 14 : 445-470).

Par ailleurs, lorsque au moins une des amorces utilisées dans le procédé d'amplification décrit plus haut comprend un segment (*b*) renfermant la séquence sens d'un promoteur pour une ARN polymérase, une activité ARN polymérase pourra être associée aux activités ADN polymérase et de déplacement de brin.

Dans un mode de réalisation particulier, l'acide nucléique de départ peut être un ADN ou un ARN extrait d'un échantillon biologique par exemple. Dans ce cas, il est possible d'obtenir une réaction d'amplification telle que définie précédemment, au départ de l'acide nucléique de départ, en une seule étape en ajoutant tous les réactifs (amorces, polymérases, etc ) dès le début de la réaction éventuellement après dénaturation de la molécule de départ. Ce mode de réalisation particulier est caractérisé par le fait que pour obtenir ledit polynucléotide utilisé comme produit de départ dans le procédé d'amplification, on opère au départ d'un acide nucléique contenant la séquence cible à amplifier et se prolongeant au-delà de l'extrémité 5' de ladite séquence cible par une région amont et au-delà de l'extrémité 3' de ladite séquence cible par une région aval. Dans ce procédé, on met en contact ledit acide nucléique de départ en présence d'un système à activité ADN polymérase ou à activités ADN polymérase et ARN polymérase, avec déplacement de brin et d'un ensemble d'amorces contenant les amorces décrites ci-dessus, respectivement, et contenant en outre une cinquième amorce capable de s'hybrider avec la région aval de l'acide nucléique de départ et/ou une sixième amorce capable de s'hybrider avec la région amont de l'acide nucléique de départ.

L'invention concerne également un ensemble d'amorces pour l'amplification cyclique d'une séquence cible d'un acide nucléique et/ou une séquence complémentaire de ladite séquence cible, ledit ensemble comprenant :
- une première amorce H telle que définie selon la présente invention,
- une seconde amorce S comprenant une séquence au moins en partie homologue du segment (*a*') de ladite amorce H.

La méthode d'amplification de l'invention peut être appliquée à tout échantillon à analyser pouvant être isolé à partir d'une matière de départ quelconque susceptible de contenir une séquence d'acide nucléique cible. Les échantillons provenant d'animaux, par exemple de mammifères, peuvent être le sang, la moelle osseuse, la lymphe, les tissus durs (foie, rate, rein, poumon, ovaires, etc.), les crachats, les fèces, l'urine. Les échantillons de départ peuvent aussi provenir de plantes, de prélèvements du sol, d' aliments, ainsi que de toute autre source suspectée contenir des organismes biologiques.

L'isolement des acides nucléiques de ces matières de départ peut être réalisé selon des procédés connus qui comprennent notamment l'utilisation de détergents conduisant à des lysats, l'utilisation d'enzymes (lysozyme, protéinase K, par exemple), le traitement aux ultrasons, l'agitation mécanique en présence de billes ou l'utilisation d'une presse de French. Dans certains cas, il peut être nécessaire de purifier les acides nucléiques extraits afin d'éliminer d'éventuels contaminants tels que des nucléases. Dans ce cas, la purification des acides nucléiques peut être réalisée par exemple par extraction au phénol-chloroforme, chromatographie, échange d'ions, électrophorèse, centrifugation à l'équilibre et/ou capture par hybridation sur un support solide.

Lorsque les acides nucléiques sont isolés, leur fragmentation sommaire peut être réalisée par des moyens tels que le traitement aux ultrasons afin d'obtenir des fragments de taille inférieure à 20 kilobases. Cette étape préliminaire, non obligatoire, permet de faciliter la dénaturation initiale dans le cas notamment d'un acide nucléique double brin. Par ailleurs la dénaturation des acides nucléiques double brin, ou des acides nucléiques simple brin présentant une forte structure secondaire, peut être réalisée par traitement thermique ou par augmentation du pH et neutralisation du milieu, afin de permettre l'hybridation des amorces selon l'invention sur la séquence cible.

Dans le cas du procédé décrit dans la figure 4, l'acide nucléique de départ, renfermant notamment la séquence cible que l'on cherche à amplifier, a été représenté sous une forme double brin ; néanmoins, hormis l'étape de dénaturation initiale, le principe demeure le même lorsque cet acide nucléique est sous une forme simple brin.

Le procédé d'amplification, selon la présente invention, d'une séquence cible d'une molécule d'acide nucléique de départ, ladite séquence cible comprenant à son extrémité 3' une séquence aval et à son extrémité 5' une séquence amont, fait intervenir :
- deux amorces de type tige-boucle selon la présente invention H1 et H2 ,
- deux amorces de déplacement D1 et D2 ,
- deux amorces de déplacement S1 et S2 .

Les amorces H1 et H2 sont construites selon les indications concernant les amorces de la présente invention décrites précédemment. Ces deux amorces H1 et H2 comprennent successivement, de 5' en 3', quatre types de segments notés (*a*)*,* (*e*)*,* (*a*') et (*b*'), respectivement. Les premier et troisième segments (*a*) et (*a*') sont de nature nucléique et leurs séquences, bien que quelconques, présentent la particularité d'être répétées et inversées l'une par rapport à l'autre, permettant ainsi l'auto-appariement du segment (*a*) avec le segment (*a*') et la formation d'une tige. Le second segment *(e),* qui est ici de nature nucléique et de séquence quelconque ne présente aucune autocomplémentarité et forme une boucle lors de l'appariement de (*a*) avec (*a*'). En outre, ce segment (*e*) possède à son extrémité 3' au moins un nucléotide modifié ou toute autre molécule permettant d'arrêter la réaction d'élongation par une polymérase d'acide nucléique. Ces séquences (*a*), (*a*') et (*e*) peuvent éventuellement être communes à H1 et H2 mais peuvent tout aussi bien être différentes. Il est évident que lesdite séquences ne doivent pas comporter d'homologie significative avec l'acide nucléique de départ et ne pas donner lieu à la formation de structures secondaires autres que la structure auto-appariée tige-boucle telle que définie ci-dessus. Le quatrième segment (*b*') des amorces H1 et H2 est de nature nucléique et de séquence différente dans l'une et l'autre des amorces. En effet, le segment (*b*') de l'amorce H1 est complémentaire de la sequence aval de la séquence cible à amplifier, tandis que le segment (*b*') de l'amorce H2 est homologue de la séquence amont de ladite séquence cible. Il faut en outre noter que lorsque les amorces H1 et H2 sont introduites dans le milieu réactionnel, elles se présentent sous leur forme la plus stable, c'est à dire sous la forme auto-appariée tige-boucle.

Les amorces de déplacement D1 et D2 ont une taille pouvant varier par exemple de 5 à 50 nucléotides, et notamment de 10 à 35 nucléotides et leur séquence est complémentaire de la séquence située, sur l'acide nucléique de départ ou sur son brin complémentaire, en aval de la séquence s'appariant avec le segment (*b*') des amorces H1 et H2, respectivement.

Les amorces de déplacement S1 et S2 sont de nature nucléique, leur taille peut varier par exemple de 2 à 30 nucléotides, et notamment de 3 à 15 nucléotides et leur séquence est homologue d'au moins une partie du segment (*a*') des amorces H1 et H2, respectivement. Les amorces S1 et S2 peuvent en outre comporter, en amont de ladite séquence, un segment nucléotidique contenant le brin sens d'un promoteur pour une ARN-polymérase, ce qui permet d'obtenir une amplification supplémentaire par formation de transcrits.

Après dénaturation de l'acide nucléique de départ, lesdites amorces H1 et D1 d'une part, et H2 et D2 d'autre part, sont hybridées audit acide nucléique dénaturé (brins I et l', respectivement) (figure 4). En présence d'une ADN polymérase présentant une activité de déplacement de brin et de dNTP, l'élongation des amorces D1 et D2 provoque le déplacement de brin et la libération dans le milieu réactionnel des simples brins d'ADN obtenus par élongation des amorces H1 et H2, respectivement. Les produits ainsi libérés (Il et Il') présentent à leur extrémité 5' la séquence correspondant soit à l'amorce H1, soit à l'amorce H2, respectivement et à leur extrémité 3' les séquences déjà présentes dans la molécule de départ permettant l'hybridation soit des amorces H2 et D2 sur le produit II, soit des amorces H1 et D1 sur le produit II'. L'élongation de chacune de ces amorces, lorsqu'elles sont hybridées, se fait avec libération des brins produits par l'élongation des amorces H1 et H2. Néanmoins, lorsque la réplication parvient au niveau de l'extrémité 5' de la matrice (respectivement Il et Il'), la polymérase recopie la séquence des amorces H1 et H2, respectivement, précédemment introduites dans ces matrices. Mais la synthèse des brins d'ADN en cours est arrêtée au niveau du ou des nucléotides modifiés, ou de toute autre molécule équivalente, introduits initialement dans le segment *(e)* des amorces H1 et H2. Les produits libérés (IV et IV') présentent donc à leur extrémité 5' la séquence complète de l'amorce H1 ou H2 et à leur extrémité 3' une séquence complémentaire du segment (*a*') de l'amorce H2 ou H1, respectivement. Comme le montre la figure 4, ces polynucléotides simple brin IV et IV' ainsi obtenus peuvent s'hybrider respectivement avec d'une part les amorces H1 et S1 (S1 étant homologue d'au moins une partie du segment (*a*') de l'amorce H1) et avec d'autre part les amorces H2 et S2 (S2 étant homologue d'au moins une partie du segment (*a*') de l'amorce H2). L'élongation de ces amorces par l'ADN polymérase, avec déplacement, aboutit aux produits IV et IV' libérés par le déplacement de brin et aux produits V et V' résultant de l'élongation des amorces S1 et S2 sur les matrices IV et IV', respectivement. Les produits IV et IV', similaires en séquence aux produits IV et IV' obtenus à l'étape précédente peuvent à nouveau s'hybrider avec les amorces S1 et H1 et S2 et H2, respectivement et conduire comme précédemment à l'obtention des mêmes produits réactionnels (IV, IV', V et V'). Les produits V et V' correspondent à une molécule double brin. Ils présentent :
- à l'une de leurs extrémités, un duplex formé par une séquence correspondant à l'amorce S1 ou S2, et son brin complémentaire,
- à l'autre extrémité, un duplex formé par une séquence correspondant à l'amorce H2 ou H1, respectivement, et du brin complémentaire des segments (*a*') et (*b*') desdites amorces.

On obtient ici les produits V et V' sous la forme représentée par VI et VI', présentant :
- à l'une de leurs extrémités, un duplex formé par l'amorce S1 ou S2 et son brin complémentaire,
- à l'autre extrémité, une structure auto-appariée tige-boucle homologue de celle présente sur les amorces H1 ou H2. Ce repliement des molécules double brin V et V', permet alors de dégager une région simple brin sur laquelle l'amorce de déplacement S2 ou S1, respectivement, peut s'hybrider.

L'élongation de l'amorce de déplacement S2 ou S1 par l'ADN polymérase s'accompagne du déplacement du brin apparié portant la structure tige-boucle. On obtient un brin déplacé correspondant à la molécule IV ou IV' précédemment décrite et une molécule VII ou VII', correspondant à la séquence cible, de l'acide nucléique de départ que l'on désire amplifier. La cyclisation du procédé apparait clairement sur la figure 4 telle que présentée. L'amplification se produit à température ambiante de manière autocatalytique jusqu'à épuisement des réactifs (enzyme, nucléotides notamment) et conduit à l'accumulation des produits VII ou VII'. Ces produits amplifiés présentent en outre, à chacune de leurs extrémités, une séquence correspondant à la co-amplification d'au moins la partie du segment (*a*') de l'amorce selon la présente invention capable d'appariement avec S1 ou S2. La présence de ce fragment co-amplifié peut notamment être avantageuse lorsque l'on souhaite isoler, purifier ou détecter la séquence cible amplifiée.

Le mélange réactionnel nécessaire à la réalisation de l'amplification selon l'invention peut notamment contenir des polyols tels que le glycérol, le sorbitol ou le polyéthylèneglycol (PEG) ou des agents dénaturants et/ou des solvants tels que le (diméthyl)formamide, le diméthylsulfoxyde (DMSO) qui peuvent permettre de réduire les réactions d'hybridation non spécifiques susceptibles d'engendrer un bruit de fond. Par ailleurs le choix de la température peut permettre de définir des conditions discriminantes d'hybridation autorisant l'hybridation spécifique des différentes amorces sur leurs cibles et le déplacement de l'équilibre thermodynamique entre la forme non auto-appariée et la forme auto-appariée tige-boucle (VI et VI') des produits tels que les molécules V et V'. Cette température peut notamment varier de 30°C à 55°C avec des réactifs enzymatiques conventionnels et de 60 à 80°C si l'on a recours à des enzymes ou des réactifs thermostables.

Le nombre d'amorces utilisées peut être avantageusement réduit si l'on choisit une structure tige commune aux amorces H1 et H2. Dans ce cas les amorces de déplacement S1 et S2 peuvent être semblables. De même, certains cas particuliers de la séquence cible à amplifier permettent de choisir une amorce de déplacement D1 semblable à l'amorce de déplacement S1 et une amorce de déplacement D2 semblable à l'amorce de déplacement S2 et alors les amorces de déplacement S1, S2, D1 et D2 peuvent être semblables. On appelle ici amorces « semblables » soit des amorces qui sont identiques, soit des amorces ayant une séquence commune (en particulier deux amorces de longueurs différentes dont la plus longue contient la séquence de la plus courte).

Afin d'augmenter l'efficacité du système d'amplification, les amorces S1 et S2 peuvent être modifiées par l'addition d'un agent intercalant fixé de manière covalente (Telser et al., 1989, J. Am. Chem.Soc. 111 : 7226-7232). Cela permet de favoriser l'hybridation des amorces de déplacement S1 et S2 tout en ne perturbant pas l'élongation de l'extrémité 3' par une polymérase. De même, des molécules peuvent être greffées à l'extrémité 5' des amorces S1 et S2, comme dans le brevet francais n° FR 91 09057 et dans la demande de brevet PCT n° WO 91 19812, décrivant le couplage d'une protéine telle que l'albumine de sérum bovin. L'association de telles molécules stériquement encombrantes sur l'extrémité 5' des amorces S1 et S2 favorise la formation de la forme auto-appariée tige-boucle au détriment de la forme non auto-appariée des amorces H1 et H2 dans les molécules V et V'.

Le figure 5 décrit une variante de la méthode d'amplification des acides nucléiques utilisant les amorces de la présente invention. Dans ce cas, une étape de transcription est ajoutée afin de produire majoritairement des molécules d'ARN. Pour ce faire, les amorces H1 et H2 selon l'invention sont construites selon le modèle décrit dans la technique exposée ci-dessus, mais contiennent en plus un segment (*b*)*,* localisé entre le segment (*b*') et le segment (*a*'), renfermant une séquence promotrice d'ARN polymérase, notamment un promoteur phagique (T7, T3 ou SP6) Conformément à la figure 5, après une série d'hybridations d'amorces et d'élongations avec déplacement de brin analogues à celles décrites à la figure 4, les molécules V et V' sont obtenues dans le milieu réactionnel. Néanmoins les amorces H1 et H2 possédant notamment un segment (*b*)*,* les produits V et V' présentent à chacune de leurs extrémités un promoteur fonctionnel, sous la forme d'un ADN double brin. En présence d'un excès de ribonucléosides triphosphates et d'une ARN polymérase correspondant audit promoteur, une réaction de transcription peut être initiée à partir desdits produits. Les ARN VI et VI' ainsi synthétisés, peuvent s'hybrider aux amorces de l'invention H2 et H1 et aux amorces de déplacement S2 et S1, respectivement. Les produits déplacés VII et VII' résultant de l'élongation des amorces H2 et H1, respectivement, peuvent s'hybrider avec les amorces de l'invention H1 et H2, respectivement. L'élongation de ces amorces par l'ADN polymérase aboutit dans les deux cas à un seul et même produit VIII. Ledit produit correspond à la séquence cible à amplifier notamment flanquée en 5' et en 3' par une région promotrice fonctionnelle, pouvant diriger la transcription de nombreux transcrits, dont la séquence correspond aux molécules d'ARN VI et VI' obtenues précédemment et qui peuvent à nouveau s'hybrider avec les amorces H2 et S2 et H1 et S1, respectivement. L'ARN issu d'une des deux voies de la méthode d'amplification décrite étant substrat pour la deuxième, et *vice et versa,* il apparait donc que la méthode selon l'invention est une technique d'amplification cyclique dont l'accumulation des produits de réaction s'effectue de manière exponentielle.

Il est évident que les séquences promotrices sur les amorces de l'invention H1 et H2, peuvent être identiques ou différentes ; néanmoins dans ce second cas deux ARN polymérases seront nécessaires dans la réaction d'amplification. De même, il est possible de réaliser une amplification d'une séquence d'acide nucléique cible en utilisant une amorce selon l'invention qui renferme une région promotrice et une seconde amorce selon l'invention qui n'en renferme pas.

La nature et la longueur des amorces utilisées dans cette méthode d'amplification, les séquences de promoteurs utilisés et les concentrations d'ARN polymérases relatives à chaque type de promoteur pourront être choisies de façon à privilégier une voie d'amplification par rapport à l'autre, de manière à obtenir préférentiellement une forme ou une autre d'ADN ou d'ARN.

Si désiré, les produits amplifiés peuvent être séparés des enzymes utilisées pour l'amplification (ADN polymérase et/ou ARN polymérase), afin d'être utilisés dans des procédés ultérieurs impliquant par exemple d'autres réactions enzymatiques, d'autres systèmes d'amplification, des méthodes de séquençage ou des méthodes de synthèse d'acides nucléiques, pour ne citer que quelques exemples.

Bien entendu, le procédé de l'invention peut être suivi par exemple d'étapes de séparation et / ou de quantification des produits d'amplification, ces étapes pouvant être mises en oeuvre de façon connue en soi.

L'invention a encore pour objet un nécessaire (amorces, enzymes) pour la détection d'un acide nucléique cible susceptible d'être présent dans un échantillon permettant la mise en oeuvre de la méthode d'amplification décrite précédemment.

L'invention a également pour objet un procédé de préparation d'un oligonucléotide utilisable notamment comme amorce dans un procédé d'amplification d'acide nucléique caractérisé par le fait que l'on synthétise, selon des méthodes connues en soi, un oligonucléotide tel qu'il a été défini dans l'invention.

Les exemples suivants illustrent l'invention sans toutefois la limiter. Sauf indication contraire, les méthodes relatives à la mise en oeuvre de ces exemples ont été réalisées conformément à leur description par Sambrook *et al.* (1989. Molecular Cloning : A Laboratory Manual, 2nd Edition, Cold Spring Harbor Laboratory, Cold Spring Harbor).

### EXEMPLE 1 :

Les oligodésoxyribonucléotides ont été obtenus par synthèse en phase solide sur un synthétiseur Applied Biosystems. La synthèse a été effectuée en utilisant les dérivés cyanoéthyl-2 N,N- diisopropylamido-phosphite des différents monomères en respectant les protocoles définis par le constructeur. Tous les réactifs ou solvants utilisés sont habituellement commercialisés.

La synthèse de la chaîne oligonucléotidique est réalisée à partir d'un support Controlled Pore Glass de porosité 1000 Å portant le nucléoside situé en 3' de la séquence. En cours de synthèse le maillon non nucléotidique (agent bloquant) est incorporé en utilisant soit l'aminoModifier Il (Clontech ref: 5203) par la suite désigné par N, soit le Spacer Phosphoramidite (Clontech ref: 5260) par la suite désigné par N'. L'élongation de la chaîne est poursuivie tout en maintenant le groupement diméthoxytrityle bloquant la fonction hydroxyle 5' du dernier nucléoside. Le clivage du support et la déprotection de l'oligonucléotide sont réalisés par un traitement avec une solution aqueuse d'ammoniac 33% pendant 12 heures. Après addition d'une solution 0,1 M d'acétate de triéthylamine et concentration du produit d'élongation sous pression réduite, une purification est réalisée par la technique HPLC en utilisant une colonne en phase inversée (RPMC C₈ 10 x 250 mm). L'élution est conduite avec un gradient d'acétonitrile (Tampon A: 0,1M acétate de triéthylamine pH: 7 ; tampon B : 50% Tampon A / 50% acétonitrile . Débit 4,7 ml/mn, de 10% de B à 50% de B en 20 mn, détection spectrophotométrique à 260 nm). Les différentes fractions positives sont combinées et concentrées sous pression réduite. Un traitement avec une solution aqueuse d'acide acétique à 80% pendant 30mn à température ambiante permet de libérer la fonction hydroxyle 5'. L'acide acétique est évaporé en présence d'éthanol. Une précipitation à l'éthanol permet d'isoler le composé désiré. Une analyse par HPLC est ensuite effectuée afin de contrôler l'intégrité des produits (colonne RPMC C₈ 4,6 x 25 mm, débit 1 ml/mn, de 10 à 14% de B en 18 mn puis de 24 à 30 % de B en 17 mn).

| SEQ ID. N° : | Structure | Temps de Rétention |
|---|---|---|
| 1 | (nucléotide)₁₄-N-N-(nucléotide)₆₀ | 23,60 |
| 2 | (nucléotide)₁₄-N'-(nucléotide)₆₀ | 21,35 |
| 3 | (nucléotide)₁₄-N-N-(nucléotide)₆₁ | 21,16 |
| 4 | (nucléotide)₁₄-N'-(nucléotide)₆₁ | 21,42 |
| 5 | (nucléotide)₁₄-N-N-(nucléotide)₃₀ | 20,38 |
| 6 | (nucléotide)₁₄-N'-(nucléotide)₃₀ | 18,55 |
| 7 | (nucléotide)₁₄-N-N-(nucléotide)₃₁ | 18,88 |
| 8 | (nucléotide)₁₄-N'-(nucléotide)₃₁ | 19,28 |

### EXEMPLE 2 :

La capacité de déplacement de brin d'une ADN polymérase sur cible ADN ou ARN en présence des amorces tige-boucle de la présente invention a été démontrée par un essai en phase homogène c'est à dire que le produit d'élongation simple brin obtenu à partir des amorces tige-boucle selon la présente invention est détecté sans étape de dénaturation, suite à son déplacement par l'élongation d'une amorce hybridée en amont sur la cible simple brin. Le modèle d'étude choisi est la séquence du gène *tem* codant pour la β-lactamase, enzyme conférant une résistance à l'antibiotique ampicilline. La séquence de ce gène est décrite dans la séquence SEQ ID n° : 9. Cette séquence est clonée dans le vecteur de clonage pBR322.
Afin d'analyser la capacité de déplacement de brin d'une ADN polymérase sur cible ARN, les ARN correspondant à la séquence du gène *tem* (SEQ ID n° : 9) ont été transcrits *in vitro,* à partir de 10¹² copies de matrice ADN double brin, par l'ARN polymérase T7, dans les conditions réactionnelles préconisées dans le kit MEGAscript™ (Ambion). Dans un premier temps, la matrice d'ADN double brin est synthétisée par la technique de la Polymerase Chain Reaction (PCR), à partir du clone pBR322, à l'aide des amorces 1588 (SEQ ID n° : 10) et 1562 (SEQ ID n° : 11). Le produit de 889 paires de bases obtenu contient notamment, à l'une de ses extrémités, le promoteur du phage T7. En présence de l'ARN polymérase T7, un ARN de 867 bases est obtenu qui correspond à la séquence complémentaire de la séquence SEQ ID n° : 9. Ces ARNs sont traités à la DNase I, purifiés par extraction au phénol/chloroforme et précipités à l'éthanol en présence de sels d'acétate d'ammonium. Les ARN sont repris dans de l'eau préalablement traitée au diéthylpyrocarbonate (DEPC), analysés par électrophorèse en gel de polyacrylamide dénaturant et dosés par absorbance à 260 nm. Une quantité équivalente à 10¹² copies d'ARN cible est utilisée par essai de déplacement de brin. Afin de réaliser les essais de déplacement de brin sur matrice ADN, un oligonucléotide de 100 bases appelé 2585 (SEQ ID n° : 12) correspondant à la région complémentaire des nucléotides 303 à 402 inclus de la SEQ ID n° : 9 a été synthétisé chimiquement et purifié par HPLC.
Les essais de déplacement sont réalisés dans un volume final de 50 µl contenant 10¹² copies de cible (ARN ou ADN), 1 mM de dATP,1 mM de dCTP,1 mM de dGTP et 1 mM de dTTP (Pharmacia) et de 5% de glycérol. Le mélange réactionnel comprend également 500nM d'une amorce oligonucléotidique 2810 (SEQ ID n° : 6) correspondant à l'amorce que l'on veut déplacer et facultativement 500nM d'une amorce de déplacement appelée DIS5 (SEQ ID n° : 13). Le mélange réactionnel est chauffé pendant 3 minutes à 65°C puis préincubé 10 minutes à 37°C. L'enzyme, soit 200 U de transcriptase inverse "Superscript^{II}" (Gibco-BRL) est ajoutée. La réaction de transcription inverse est réalisée pendant une heure à 37°C, puis on congèle à -20°C. Une fraction de 5 µl est analysée, sans étape de dénaturation, selon la méthode modifiée de capture et détection spécifique ELOSA (Enzyme Linked Oligo Sorbent Assay). Cette méthode fait intervenir la fixation d'un oligonucléotide de capture sur support solide (plaque de microtitration), l'hybridation du produit déplacé-élongé lors de la réaction sur une sonde de capture spécifique dudit produit et sa révélation par une sonde de détection couplée à la peroxydase de raifort. L'oligonucléotide de capture A25 (SEQ ID n° : 14) est fixé de façon passive dans les puits d'une plaque de microtitration Nunc-lmmuno plate (Maxisorp) selon la méthode déjà décrite dans le brevet Français n°91 09057, permettant la fixation d'environ 5 pmoles d'oligonucléotide sur un puits. Après fixation, trois lavages avec du tampon PBS Tween 1X sont effectués. La détection du produit de déplacement capturé est réalisée conformément à la technique décrite dans le brevet Français n° 91 09057. La fraction de 5 µl à analyser est ajoutée à un volume de 45 µl de tampon phosphate de sodium 0,2 M pH 7, chlorure de sodium 1 M, EDTA 2 mM, SDS 1,3%, ADN de saumon 0,24 mg/ml, polyéthylène glycol (PEG) 6000 4%. Chaque échantillon est déposé dans le puits d'une plaque de microtitration traité avec la sonde de capture A25 (SEQ ID n° : 14), comme décrit précédemment. Immédiatement après, 50 µl 1 de tampon phosphate de sodium 0,2 M pH 7, chlorure de sodium M, EDTA 2 mM, SDS 1,3%, ADN de saumon 0,24 mg/ml, polyéthylène glycol (PEG) 6000 4%, contenant 5 ng de sonde oligonucléotidique de détection A28 (SEQ ID n° : 15) couplée à la peroxydase de raifort sont ajoutés. Après incubation 60 minutes à 37°C, les puits sont lavés au PBS Tween 1X. La révélation de la sonde A28-peroxydase hybridée sur le produit déplacé est effectuée par addition de 100 µl d'une solution contenant le substrat ortho-phenylène diamine (OPD). La réaction colorimétrique est arrêtée après 20 minutes par addition de 100 µl d'acide sulfurique 1 M. La densité optique à 492 nm est lue grâce à un lecteur AXIA microreader (bioMérieux).
Les résultats obtenus sont représentés sur la figure 6. Ils montrent d'une part que la présence d'une amorce de déplacement (SEQ ID n°13) permet d'obtenir une libération conséquente, dans le milieu, du brin issu de l'élongation de l'amorce déplacée (oligonucléotide 2810), que ce soit sur matrice ADN ou ARN. En absence d'amorce de déplacement, le signal correspondant à l'élongation de l'amorce 2810 est environ 2 fois plus faible. Ceci démontre l'efficacité du déplacement de brin sur matrice ARN et ADN à l'aide de la transcriptase inverse Superscript^{II}™. On peut en outre quantifier ces résultats, ce qui permet de montrer que cette transcriptase inverse; dépourvue d'activité RNase H, possède une meilleure efficacité de déplacement d'un brin ADN sur matrice ARN que sur matrice ADN.

### EXEMPLE 3 :

Cet exemple est destiné à montrer que des matrices renfermant une structure auto-appariée tige-boucle en amont d'une séquence promotrice peuvent être transcrites par une ARN polymérase. Ce type de matrice a été synthétisé, à partir de pBR322, par la technique PCR à l'aide des oligonucléotides 2801 (SEQ ID n° : 1) et 1028 (SEQ ID n° : 18), d'une part et 2806 (SEQ ID n° : 2) et 1028 (SEQ ID n° : 18), d'autre part, en présence du fragment de Stoffel de la *Taq* polymérase (Applied Biosystems), dépourvu d'activité exonucléase 5'-3'. L'absence de cette activité exonucléase permet de s'affranchir de la dégradation éventuelle de la partie tige des structures tige-boucle de la présente invention lors des différents cycles de PCR nécessaires à la synthèse desdites matrices. Les produits PCR obtenus (matrices), analysés sur gel d'agarose Nusieve 3% (FMC), possèdent un poids moléculaire apparent de 130 paires de bases. Ils contiennent, en aval d'une structure auto-appariée tige-boucle, le promoteur de l'ARN polymérase du phage T7 (SEQ ID n° : 19). Les bandes correspondant à ces produits sont découpées du gel d'agarose et les produits PCR sont électroélués. Ils sont précipités à l'éthanol en présence d'acétate de sodium et dosés à 260 nm. Les essais de transcription sont réalisés à 37°C pendant deux heures dans un volume de 50 µl final, dans le tampon de réaction décrit par Milligan *et al.* (1987. *Nucl. Acids Res.* 15 : 8783-8798), en présence de ATP, CTP, GTP et UTP (4 mM chaque, Boehringer), 1 U/µl de RNA guard (Pharmacia), 1 U/µl d'ARN polymérase T7 (New England Biolabs) et de 10¹¹ copies de matrice PCR purifiée.
Une fraction du mélange réactionnel (10 µl) est mélangée à 10 µl d'une solution 0,035 % xylène cyanol, 0,035 % bleu de bromophenol, 1 mM EDTA, 98 % formamide avant d'être analysée par électrophorèse en gel de polyacrylamide dénaturant 15 % d'acrylamide, urée 7 M, 0,26 % bisacrylamide, 90 mM Tris-borate, 2 mM EDTA, pH 8,3. Ces gels sont coulés dans des appareils d'électrophorèse mini-protean Il electrophoresis cell™ (Biorad) et ont une épaisseur de 1 mm. Avant dépôt sur gel, les échantillons sont dénaturés à 65°C pendant 2 minutes et refroidis rapidement sur glace. L'électrophorèse est effectuée à 150 Volts, jusqu'à ce que le bleu de bromophénol migre à 1 cm du bas du gel. Les gels sont colorés pendant 10 minutes dans une solution de bromure d'ethidium à 0,6 µg/ml et photographiés sur une table UV (312 nm) grâce à un appareil MP4 (Polaroïd).
Les produits séparés par électrophorèse sont transférés sur membrane de nylon Hybond N™ (Amersham) grâce à un appareil mini trans-blot electrophoretic transfert cell™ (Biorad), dans un tampon 45 mM Tris-Borate, 1 mM EDTA, pH 8,3, à 4°C, sous un champ électrique de 35 Volts. heures⁻¹. cm⁻¹ . Les membranes sont séchées durant 5 minutes à 80°C et les acides nucléiques fixés sur la membrane par exposition aux rayonnements UV (312 nm) durant 3 minutes.
Ces membranes sont préhybridées à 37°C pendant 60 minutes dans 4 ml de tampon phosphate de sodium 0,1 M pH 7,0, chlorure de sodium 0,5 M, EDTA 1 mM, SDS 0,65 %, ADN de saumon 0,14 mg/ml, et polyéthylène glycol 6000 (PEG) 2 %. L'hybridation est effectuée par incubation durant 60 minutes à 37°C dans 5 ml du même tampon contenant, à la concentration de 200 ng/ml l'oligonucléotide A28 (SEQ ID n° : 15) marqué à la peroxydase de raifort par couplage en 5' selon le procédé précédemment décrit dans le brevet International n° WO 91/19812. Après 3 lavages de 30 secondes dans 50 ml de tampon PBS 1X (Sambrook *et al.,* 1989. Molecular Cloning : A Laboratory Manual, 2nd Edition, Cold Spring Harbor Laboratory, Cold Spring Harbor) contenant 0,5 ‰ de Tween 20, l'oligonucléotide hybridé est révélé par l'activité de la peroxydase en présence de 10 mg de tetrahydrochlorure de diaminobenzidine dihydraté (DAB) dans 20 ml de tampon phosphate de sodium 20 mM pH 7,2, chlorure de sodium 150 mM, albumine de sérum de bovin 2 mg/ml. Après incubation à température ambiante et à l'abri de la lumière pendant 15 minutes, la réaction est arrêtée par rinçage à l'eau distillée.

Un témoin d'activité de l'ARN polymérase T7 a été préalablement constitué en préparant un fragment d'ADN double brin par PCR, à partir de la cible pBR322, à l'aide des amorces A24 (SEQ ID n° : 17) et 1028 (SEQ ID n° : 18). Le produit de 285 paires de bases ainsi obtenu contient à une extrémité le promoteur du phage T7 et sa transcription par l'ARN polymérase T7 permet d'obtenir un ARN de 263 bases. Le témoin d'activité de l'ARN polymérase T7 est réalisé par incubation de 10¹¹ copies du fragment de 285 paires de bases dans les conditions précédemment décrites. Cette réaction est effectuée pendant deux heures à 37°C, en parallèle des essais de transcription sur les matrices contenant les structures tige-boucle de la présente invention.

Les résultats obtenus confirment la capacité de matrices contenant une structure auto-appariée tige-boucle en amont d'un promoteur d'ARN polymérase a être transcrites *in vitro.* La nature de la boucle ne semble pas avoir d'influence puisque les matrices contenant une boucle avec deux résidus aminoModifier II™ (amine incluse dans l'amorce 2801; SEQ ID n° : 1) ou un résidu Spacer Phosphoramidite™ (polyéthylèneglycol inclus dans l'amorce 2806; SEQ ID n° : 2) permettent d'obtenir le transcrit de taille attendue avec un rendement identique dans les deux cas.

### EXEMPLE 4 :

Les amorces tige-boucle de la présente invention doivent être recopiables partiellement par une polymérase des acides nucléiques. Dans ce but, plusieurs ADN polymérases ont été essayées : des enzymes thermostables telles que la *Taq* polymérase (Applied BioSystems), le fragment de Stoffel de la *Taq* polymérase (Applied BioSystems), la *Bca* polymérase (Takara), la *Vent™* polymérase (New England Biolabs), et des enzymes thermolabiles telles que le fragment de Klenow (Boehringer), la transcriptase inverse Superscript™ (Gibco-BRL) et la T4 ADN polymérase (New England Biolabs). Ces polymérases ont été testées vis à vis de leur capacité à élonger une amorce sur une matrice constituée par des oligonucléotides tige-boucle selon la présente invention, ainsi que sur une matrice témoin 2799 (SEQ ID n° : 21) ne comportant pas de structure de ce type. L'amorce A26 (SEQ ID n° : 22), complémentaire de l'extrémité 3' de ces matrices, a été marquée par l'isotope ³²P à son extrémité 5' par la T4 polynucléotide kinase en présence de [g-³²P] ATP. L'amorce marquée a été purifiée par filtration sur gel Séphadex G25 (Pharmacia) et l'équivalent de 10 pmoles d'amorce marquée, correspondant à 2 x10⁷ cpm, est hybridé à 20 pmoles de matrice, dans le tampon commercial correspondant à l'enzyme (volume final 20 ml) L'enzyme est ajoutée (3 U de T4 ADN polymerase, ou 200 U de transcriptase inverse, ou 5 U de klenow, ou 1 U de polymérase thermostable) puis une incubation d'une heure est réalisée à 37°C pour les enzymes thermophiles et à 50 °C pour les enzymes thermostables. Les réactions sont arrêtées par congélation à -20°C. Les produits sont ensuite analysés par électrophorèse en gel d'acrylamide dénaturant. Les gels sont formés par polymérisation d'une solution contenant 20 % d'acrylamide, urée 7 M, 0,26 % bisacrylamide, 90 mM Tris-borate, 2 mM EDTA, pH 8,3 et sont coulés dans des appareils d'électrophorèse The Sturdier™ (Hoefer Scientific Instruments) d'une épaisseur de 1 mm. Les échantillons à analyser sont préparés par mélange de 10 µl de mélange réactionnel et de 10 µl d'une solution 0,035 % xylène cyanol, 0,035 % bleu de bromophenol, 1 mM EDTA, 98 % formamide. Avant dépôt sur gel, ces échantillons sont dénaturés à 95°C pendant 2 minutes, puis refroidis rapidement sur glace. L'électrophorèse est effectuée à 450 Volts, jusqu'à ce que le bleu de bromophénol migre à 1 cm du bas du gel. Les gels sont séchés sur papier Whatman 3MM et autoradiographiés pendant 2 heures. La taille des produits d'extension est déterminée par rapport à un marqueur de poids moléculaire constitué de différents oligonucléotides marqués en 5' comme les amorces, déposé en parallèle sur le gel.

L'analyse montre une élongation complète de l'amorce sur la matrice témoin SEQ ID N°21, en présence des différents types d'enzymes utilisées, conduisant à un produit d'extension de 59 bases. Sur les matrices 2801 (SEQ ID N°1) et 2804 (SEQ ID N°21), des produit d'élongation de 60 et 30 bases sont obtenus, respectivement, en présence des enzymes Stoffel, Superscript™, *Vent*™ et Klenow. Ces produits correspondent à une élongation des amorces sur les cibles comprenant les structures auto-appariée tige-boucle jusqu'à la dernière base précédant la modification chimique aminoModifier II^{''''}™. Ceci démontre d'une part que la structure auto-appariée tige-boucle ainsi formée peut être ouverte grace à l'activité de déplacement de brin de ces enzymes, et que ces structures auto-appariée tige-boucle ne sont que partiellement recopiables du fait de la présence de la modification chimique car la réplication de ces structures est arrêtée par ladite modification. De même, des résultats de même nature sont obtenus sur les matrices 2806 (SEQ ID N°2) et 2810 (SEQ ID N°6) puisque des produit d'élongation de 60 et 30 bases sont obtenus, respectivement, en présence des enzymes Stoffel, Superscript^{II}™, *Vent*™ et Klenow. Ces produits correspondent à l'élongation de l'amorce A26, sur des cibles comprenant des structures auto-appariée tige-boucle, jusqu'à la dernière base précédant la modification chimique Spacer Phosphoramidite™. L'utilisation des enzymes *Taq* et *Bca* ne permet pas d'obtenir les produits d'extension attendus, quel que soit le type structure tige-boucle utilisé. Ces enzymes possédant une activité exonucléase (5'-3'), il est probable qu'elles dégradent la matrice avant même d'avoir pu la recopier. Il est utile de noter, dans le cas où on utilise la T4 ADN polymérase, qu'aucun produit d'extension n'est observé en présence des matrices 2804 et 2810, et que par contre, un produit d'extension d'environ 50 nucléotides est obtenu en présence des matrices 2801 et 2806. Ceci semble indiquer que la T4 ADN polymérase n'est pas capable d'ouvrir, afin de les copier, les structures tige boucle utilisées dans le présent exemple.

### EXEMPLE 5 :

On réalise une amplification selon la méthode décrite à la figure 5. Pour ce faire un fragment de 167 paires de bases a été synthétisé par la technique PCR à l'aide des amorces DTA7 (SEQ ID n° : 23) et DTA8 (SEQ ID n° : 24), à partir de pBR322, et qui contient donc une partie de la séquence du gène *tem* correspondant aux nucléotides 336 à 402 inclus de la SEQ ID n° : 9. Ce fragment contient à chacune de ses extrémités une séquence promotrice d'ARN polymérase (en particulier le promoteur du phage T7), précédée en 5' par une séquence homologue à la séquence E220 (SEQ ID n° : 25). Afin de mettre en évidence le caractère exponentiel de la méthode, différentes réactions d'amplification ont été menées en parallèle en présence de quantités décroissantes de molécule cible constituée par le fragment de PCR précédent purifié : de 10¹¹ à 10⁷ copies par essai. Les réactions sont réalisées dans un volume final de 50 µl, en présence d'ATP, CTP, GTP et UTP (4 mM chaque), de dATP, dCTP, dGTP et dTTP (1 mM chaque) de 1 U/µl d'ARN polymérase du phage T7 (New England Biolabs), de 4 U/µl de Transcriptase inverse MMLV Superscript^{II}™ (Gibco-BRL), dépourvue d'activité RNase H, et 1 U/µl de RNA guard (Pharmacia). Le milieu réactionnel contient en outre les amorces H1 et H2 selon l'invention correspondant aux oligonucléotides 2806 et 2808 (SEQ ID n° : 2 et SEQ ID n° : 4, respectivement) à une concentration de 1 µM et les amorces de déplacement E220 (SEQ ID n° : 25) et S1 (ou S2) correspondant à l'oligonucléotide DIS8 (SEQ ID n° : 26) à une concentration de 0,1µM. Après deux heures d'incubation 37°C, les réactions d'amplification sont arrêtées par congélation du milieu réactionnel à -20°C. Une fraction de 5 µl, soit 1/10 de volume réactionnel est analysée quantitativement par capture et détection spécifique selon la méthode ELOSA (Enzyme Linked Oligo Sorbent Assay). La méthode fait intervenir la fixation d'un oligonucléotide de capture sur support solide (plaque de microtitration), la dénaturation du produit de réaction, son hybridation sur la sonde de capture spécifique de la séquence amplifiée et la révélation par une sonde de détection couplée à la péroxydase de raifort. L'oligonucléotide de capture A20 (SEQ ID n° : 27) est fixé de façon passive sur les puits d'une plaque de microtitration Nunc-lmmuno plate de Maxisorp selon la méthode déjà décrite dans le brevet Français n°9109057, permettant la fixation d'environ 5 pmoles d' oligonucléotide sur un puits. Après fixation, trois lavages avec du tampon PBS Tween 1X sont effectués. La détection du produit d'amplification capturé est réalisée conformément à la technique précédemment décrite dans le brevet Français n° 91 09057. La fraction de 5 µl à analyser est ajoutée à un volume de 35 µl de tampon phosphate de sodium 0,2 M pH 7,0, chlorure de sodium 1 M, EDTA 2 mM, SDS 1,3%, ADN de saumon 0,24 mg/ml, polyéthylène glycol (PEG) 6000 4%. Les acides nucléiques contenus dans cet échantillon sont dénaturés par addition de 5 µl d'hydroxyde de sodium 2 M à température ambiante, et neutralisés après 3 minutes par addition de 5 µl d'acide acétique 2 M. Pour contrôle et étalonnage, deux gammes de dilution d'ARN correspondant aux produits d'amplification attendus sont effectuées. Chaque échantillon est alors déposé dans le puits d'une plaque de microtitration dans lequel a été préalablement fixée la sonde oligonucléotidique de capture A20 (SEQ ID n° : 27). On dépose soit 50 µl d'échantillon non dilué, soit le même volume d'échantillon dilué au 10^{ème} ou au 100^{ème}. Immédiatement, un volume de 50 µl de tampon phosphate de sodium 0,2 M pH 7,0, chlorure de sodium 1 M, EDTA 2 mM, SDS 1,3%, ADN de saumon 0,24 mg/ml, polyéthylène glycol (PEG) 6000 4%, contenant 5 ng de sonde oligonucléotidique de détection A 19 (SEQ ID n° : 28) couplée à la peroxydase de raifort, est ajouté. Après incubation 60 minutes à 37°C, les puits sont lavés au PBS Tween 1X. La révélation de la sonde A19-péroxydase hybridée sur le produit d'amplification est effectuée par addition de 100 µl d'une solution contenant le substrat ortho-phenylène diamine (OPD). La réaction colorimétrique est arrêtée après 20 minutes par addition de 100 µl d'acide sulfurique 1 M. La densité optique à 492 nm est lue grâce à un lecteur AXIA microreader (bioMérieux). Les résultats obtenus sont représentés par les histogrammes dans la figure 7. Pour chacune des dilutions de cible, deux essais ont été réalisés : essai complet (comme décrit précédemment) et essai sans amorce de déplacement E220 et DIS8. Les résultats montrent que la méthode d'amplification (système complet) permet de détecter dans ces conditions un signal spécifique significatif jusqu'à une quantité de cible initiale de 10⁸ copies par essai, soit une sensibilité de 10⁷ copies, puisqu'une fraction équivalant à 1/10^{ème} du milieu réactionnel est analysée dans ces conditions. Cette sensibilité n'est que relative et peut être grandement améliorée si l'on utilise un système de révélation autre que la colorimétrie (par exemple la fluorescence, la chemiluminescence ou la bioluminescence). Les résultats montrent surtout qu'en absence d'amorce de déplacement E220 et DIS8, un signal spécifique n'est obtenu que pour une quantité de cible égale 10⁹ copies par essai. La différence de sensibilité de détection entre "avec" et "sans" amorce de déplacement est donc d'un facteur 10², ou de 2 unités Log de base 10. Ceci démontre que la méthode permet une accumulation importante de produit d'amplification, celle-ci ne pouvant provenir que de la réalisation du cycle d'amplification. La réalisation du cycle n'est donc possible qu'en présence des amorces de déplacement. Ces données montrent que la méthode repose sur la cyclisation du procédé jusqu'à l'étape de transcription par le biais d'une étape de déplacement enzymatique, en particulier à l'aide d'une amorce de déplacement en présence d'une ADN polymérase telle qu'une transcriptase inverse. L'analyse qualitative des essais d'amplification par séparation électrophorétique, transfert sur membrane de nylon et hybridation, comme décrit dans l'exemple 3, à l'aide de la sonde A28 (SEQ ID n° : 15) d'une part et de la sonde A19 (SEQ ID n° : 28) d'autre part couplées à la peroxydase de raifort, a permis de mettre en évidence deux molécules d'ARN complémentaires de 110 bases, correspondant au produit d'amplification attendu.

### EXEMPLE 6:

Afin de démontrer la capacité de blocage de la polymérisation par différents bras non nucléotidiques, plusieurs types d'amorces tige-boucle ont été synthétisées (selon la technique décrite dans l'exemple 1) : les unes renferment un bras non nucléotidique Aminomodifier II™ (Clontech ref: 5203) comportant 2 (3116 ; SEQ ID n° : 33) ou 4 carbones (2804 ; SEQ ID n° : 5), les autres un bras Spacer Phosphoramidite™ (Clontech ref: 5260) à 12 carbones (2810 ; SEQ ID n° : 6) ou un nucléoside α-thymidine, noté αdT, 3531 (SEQ ID n° : 32) (le phosphoramidite nécessaire à l'incorporation du nucléoside est préparé à partir du nucléoside commercial Sigma ref: T3763). L'arrêt de la polymérisation le long de ces structures tige-boucle particulières a été analysé avec plusieurs polymérases:a) des enzymes thermostables telles que le fragment de Stoffel de la *Taq* polymérase (Applied biosystems) ou la 9°Nm™ (New England Biolabs) et b) des enzymes thermolabiles telles que le fragment de Klenow de la polymérase I *d'Escherichia coli* (Boehringer) ou la transcriptase inverse Superscript II™ (Gibco BRL).

Dans un premier temps, l'amorce A26 (SEQ ID n° : 22) qui est complémentaire de l'extrémité 3' de chacune des matrices, a été marquée à son extrémité 5' par la T4 polynucléotide kinase en présence de [γ-³²P]ATP, puis purifiée par filtration sur gel Sephadex G-25 (Pharmacia). Dans un second temps, 10¹² copies d'amorces tige-boucle (SEQ ID n°33, 5, 6, ou 32) et 10¹² copies d'oligonucléotide A26 (SEQ ID n° : 22 ) marqué sont mélangés en présence de chacun des dNTPs (1 mM final) dans un volume réactionnel de 20 µl (tampon commercial (1X final) correspondant à chacune des enzymes que l'on souhaite utiliser) et en présence de 200U de transcriptase inverse, de 50U du fragment de Klenow, de 1U du fragment de Stoffel de la *Taq* polymérase, ou de 2U de la 9°Nm. Après une incubation pendant une heure à 37°C, les réactions sont arrêtées par congélation à -20°C et les produits sont analysés par électrophorèse en gel de polyacrylamide dénaturant, comme décrit dans l'exemple 4. La taille des produits d'élongation est déterminée par rapport à un marqueur de poids moléculaire constitué de différents oligonucléotides marqués en 5' et déposés en parallèle sur le gel.

Les résultats obtenus montrent que l'élongation de l'amorce A26 est interrompue par le bras non nucléotidique sur les matrices constituées de la structure tige-boucle 2804 (SEQ ID n° : 5), 2810 (SEQ ID n° : 6), 3239 (SEQ ID n° : 34) et 3531(SEQ ID n° : 32) lorsque la réplication est effectuée avec le fragment de Klenow ; sur les matrices constituées de la structure tige-boucle 2810 (SEQ ID n° : 6), 3239 (SEQ ID n° : 34) et 3531(SEQ ID n° : 32) lorsque la réplication est effectuée avec la transcriptase inverse ; sur les matrices constituées de la structure tige-boucle 2804 (SEQ ID n° : 5), 2810 (SEQ ID n° : 6), 3116 (SEQ ID n° : 33), 3239 (SEQ ID n° : 34) et 3531(SEQ ID n° : 32) lorsque la réplication est effectuée avec le fragment de Stoffel. L'utilisation de structures tige-boucle comportant un bras non nucléotidique polyéthylèneglycol de 12 carbones (Spacer Phosphoramidite), par exemple les structures 2810 (SEQ ID n° : 6) et 3239 (SEQ ID n° : 34) est une solution avantageuse pour la réalisation de la présente invention car elle ne permet pas de polymérisation au-delà du site non nucléotidique, quelle que soit l'enzyme utilisée. Il semble en outre qu'il existe une relation entre la longueur et/ou la nature du bras non nucléotidique et la capacité d'arrêt de la polymérase par cet élément par une enzyme donnée.

### EXEMPLE 7:

Afin de démontrer la capacité d'un fragment d'ADN double brin à former une structure tige-boucle selon l'invention, un fragment d'ADN double brin de 135 paires de bases a été synthétisé. Pour cela, une partie du gène *tem* (SEQ ID n° : 9) inclus dans le plasmide pBR322, a été amplifié par la technique de PCR à l'aide de l'amorce tige-boucle 3336 (SEQ ID n° : 35), qui renferme un bras non nucléotidique de type AminoModifer II™, et de l'amorce 1863 (SEQ ID n° : 39) en présence du fragment de Stoffel de la *Taq* polymérase (Applied Biosystems) dépourvue d'activité exonucléase 5'-3'. Le fragment obtenu contient, à l'une des extrémités 5', une séquence susceptible de former une structure tige-boucle. En parallèle, une PCR de contrôle est réalisée en présence de l'amorce 3215 (SEQ ID n° : 43) et de l'amorce 1863 (SEQ ID n° : 39). Le produit d'amplification par PCR ainsi obtenu renferme seulement la séquence de la tige et constitue un témoin de poids moléculaire de 135 paires de bases sous forme duplex.

Après PCR, les produits sont analysés sur gel d'agarose SeaKem LE 2% (FMC). Après migration à 80 V pendant 2 heures dans une solution de tampon TBE 1X (89 mM Tris-HCI pH 8,3 89 mM acide borique, 2 mM EDTA), le gel est coloré en présence de bromure d'ethidium (0,6 µg/ml). L'analyse des produits de PCR met en évidence deux bandes de taille distincte correspondant à deux formes de migration des produits obtenus à partir de l'amorce tige-boucle 3336 (SEQ ID n° : 35) et une seule forme de migration dans le cas du témoin de poids moléculaire. Les deux formes de mobilité électrophorétique visualisent deux conformations différentes, tige-boucle ou duplex, de l'une des extrémités 5' du fragment d'ADN. La forme dont la migration électrophorétique est la plus rapide correspond à une molécule au sein de laquelle la séquence de l'amorce 3336 introduite dans le fragment PCR se trouve sous une forme duplex, la forme dont la migration électrophorétique est la plus lente correspond à une molécule au sein de laquelle ladite séquence de l'amorce 3336 est repliée sous la forme d'une tige-boucle.

Les bandes observées sur gel d'agarose sont découpées et purifiées à l'aide d'un kit d'extraction sur gel Qiaex II™ (Qiagen, ref : 20021). Les produits purifiés sont déposés sur gel d'agarose SeaKem LE 2% (FMC) dans des conditions identiques à celles décrites plus haut. La migration du produit correspondant à la forme rapide se traduit par une seule bande migrant de manière identique à celle issue de la purification du produit de la PCR de contrôle. Le produit correspondant à la forme lente précédemment isolée conduit à l'obtention d'une seule bande retardée par rapport à celle issue de la purification de la PCR de contrôle.

Afin de confirmer que les deux bandes obtenues sur gel d'agarose correspondent à deux formes isomères du même produit d'amplification par PCR, chacune des bandes isolées et purifiées précédemment à partir du produit de cette PCR ont été séquencée à l'aide d'un kit Dye Deoxy Terminator cycle sequencing (Applied Biosystems). La réaction de séquençage nécessite une première amplification réalisée à partir 10¹¹ copies du produit purifié, en présence de 10 µl de mélange réactionnel de séquençage et de 5 µl de l'amorce 1863 (SEQ ID n° : 39) de la PCR initiale. La réaction de séquençage est réalisée sur un appareil Thermocycler 480 (Perkin Elmer) selon le cycle suivant: 30 secondes à 96°C, 15 secondes à 40°C et 4 minutes à 60°C, l'ensemble répété 25 fois. Ce produit PCR est ensuite purifié sur colonne G-50 Sephadex (Pharmacia), puis séquencé sur un séquenceur automatique (Applied Biosystems 373 A).

L'analyse des résultats montre qu'il n'existe pas de différence entre les séquences obtenues à partir du produit de la PCR de contrôle, de la forme rapide ou de la forme lente purifiées précédemment. De plus, la conformité de séquence entre, d'une part, le produit de la PCR de contôle et, d'autre part les deux formes isomères isolées confirme que la partie tige de la structure tige-boucle est répliquée de manière fidèle par le fragment de Stoffel de la *Taq* polymérase. Cette réplication est interrompue, comme précédemment démontré dans l'exemple 6, par la partie non nucléotidique de la boucle des amorces de la présente invention.

L'ensemble de ces éléments montrent que l'incorporation d'une amorce capable de former une structure secondaire de type tige-boucle, selon la présente invention, permet la formation de deux formes isomères comportant à leur extrémité une même séquence soit sous la forme d'une tige-boucle soit sous la forme d'un duplex.

### EXEMPLE 8:

Des essais de déplacement (selon le modèle de la Figure 9) ont été réalisés sur un ARN transcrit à partir du produit d'amplification par PCR de 572 paires de bases préalablement obtenu avec les amorces 2434 (SEQ ID n° : 40) contenant un promoteur du phage T7 à son extrémité 5', et 3239 (SEQ ID n° : 34) possédant un résidu Spacer Phosphoramidite™ (hexaéthylèneglycol). Cet ARN possède à son extrémité 3' une séquence complémentaire de la partie tige de l'amorce 3239, permettant l'hybridation d'une amorce de déplacement Dis 22 [γ-³²P]ATP (SEQ ID n° : 44). Cet ARN peut en outre s'hybrider avec l'amorce tige-boucle A21 (SEQ ID n° : 41) à son extrémité 3'. Un témoin réactionnel est réalisé en substituant l'amorce tige-boucle 3239 par l'amorce 3215 (SEQ ID n° : 43) qui possède la séquence de tige en amont (5') de la séquence A21. Les réactions de déplacement sont réalisées dans un volume final de 20 µl en présence de transcriptase inverse Superscript II™ (GIBCO-BRL) dans le tampon optimisé par le fournisseur, de dNTPs (1 mM chaque, Pharmacia), de 10¹¹ copies de cible par essai et de 5 x 10¹² copies d'amorce par essai. Après une incubation pendant 30 minutes à 37°C, les réactions sont arrêtées par adjonction de 20 µl de tampon 0,02% Xylène Cyanol, 0,02% Bleu de Bromophénol, 25 mM EDTA, 90% formamide. Les échantillons sont dénaturés à 65°C pendant 3 minutes, refroidis rapidement sur glace et analysés par électrophorèse en gel de polyacrylamide 8% dénaturant (7 M urée) en tampon TBE (Tris-borate 90mM, EDTA 2mM, pH 8,3). L'électrophorèse est effectuée à 300 V jusqu'à ce que le front de migration parvienne à environ 1 cm du bas du gel.

Un contrôle de l'élongation de l'amorce tige-boucle 3239 (SEQ ID n° : 34) par la transcriptase inverse sur la matrice ARN, en présence de l'amorce de déplacement Dis 22 (SEQ ID n° : 44) est réalisé dans lequel l'amorce 3239 (SEQ ID.n° : 34) marquée en 5' par du [γ-³²P]ATP est incubée, dans les conditions exposées plus haut, en présence de l'amorce de déplacement Dis 22 (SEQ ID n° : 44) non marquée. L'électrophorèse confirme que la structure tige-boucle est recopiée jusqu'à l'extémité 5' de la matrice ARN. De même, l'hybridation et l'élongation de l'amorce de déplacement Dis 22 (SEQ ID n° : 44) sur la matrice ARN synthétisée est vérifiée par incubation de cette amorce marquée en 5' par du [γ-³²P]ATP. Cette élongation permet la synthèse d'un ADNc dont la longueur est compatible avec la matrice ARN. Par ailleurs, l'intensité des produits visualisés reflète le degré de repliement de l'une des extrémités 5' des molécules produites, sous une forme tige-boucle.

Les essais de déplacement révèlent un produit d'élongation de l'amorce de déplacement sur matrice ARN, confirmant le déplacement du brin issu de l'élongation de l'amorce tige-boucle utilisée. Cela tend à prouver que l'amorce tige-boucle utilisée, lorsqu'elle est hybridée sur la matrice ARN, est présente sous une forme repliée tige-boucle, permettant ainsi à l'amorce de déplacement de s'hybrider sur la partie 3' de l'ARN. Lorsque les essais de déplacement sont réalisés en remplaçant l'amorce 3239 (tige-boucle) par l'amorce 3215 (absence de structure tige-boucle), la faible intensité du produit d'élongation de l'amorce de déplacement confirme que la séquence de tige contenue dans l'amorce 3215 empêche l'hybridation de l'amorce de déplacement, par un phénomène de compétition au niveau du même site d'hybridation. L'amorce tige-boucle étudiée dans ce cas particulier montre que la stabilité de cette structure tige boucle est supérieure à celle de l'hétéroduplex ADN/ARN correspondant qui pourrait se former par déplacement de l'équilibre thermodynamique. Ces résultats mettent en évidence la possibilité d'utilisation des amorces tige-boucle selon la présente invention sur une matrice ARN.

L'effet d'un agent intercalant de type acridine fixé à l'extrémité 5' d'une amorce tige-boucle sur l'efficacité de déplacement de brin sur matrice ARN a également été étudié. Dans ce but, une matrice ARN a été transcrite à partir d'un produit de PCR contenant l'amorce 3215 (SEQ ID n° : 43) et l'amorce promotrice 2709 (SEQ ID n° : 42). Les essais de déplacement sont réalisés sur matrice ARN comme précédemment décrit, en présence d'amorce de déplacement Dis 22 (SEQ ID n° : 44) marquée en 5' par du [γ-³²P]ATP.

Les essais sont conduits selon deux modes différents. selon un premier mode, l'ADNc issu de l'élongation des amorces tige-boucle étudiées est préformé en présence des réactifs adéquats (transcriptase inverse, nucléotides), et l'amorce de déplacement est ajoutée au mélange réactionnel avec un décalage de 15 minutes ; selon un deuxième mode, l'analyse est réalisée, comme précédemment, avec tous les réactifs présents dans le milieu réactionnel en même temps. Dans les deux modes, les produits réactionnels sont mis en incubation pendant 30 minutes à 37°C.

Deux amorces tige-boucle ont été étudiées : 3221 (SEQ ID n° : 36) et 3516 (SEQ ID n° : 38), cette dernière correspondant à l'amorce 3221 avec un goupement acridine (6- chloro-2-méthoxyacridine -Clontech ref 5236) situé à son extrémité 5'. Un témoin de déplacement est constitué par l'amorce A21 (SEQ ID n° : 41). Les amorces, sans aucune distinction, sont à une concentration de 5 x 10¹² copies par essai, et la matrice ARN utilisée est à 10¹¹ copies par essai. L'analyse des produits est réalisée par électrophorèse en gel de polyacrylamide 8% dénaturant tel que décrit précédemment. Des produits d'élongation de même intensité sont obtenus à partir du témoin réalisé avec l'amorce A21, avec ou sans étape de préformation, confirmant l'absence de compétition au niveau de ce système. La réalisation de l'étape de préformation dans le cas de l'amorce tige-boucle 3221 (SEQ ID n° : 36) ne conduit pas à l'obtention d'un produit d'élongation de l'amorce de déplacement, tandis que le même essai réalisé sans étape de préformation donne un résultat positif comme précédemment décrit. Des signaux de même intensité sont obtenus en utilisant l'amorce 3516 (SEQ ID n° : 38), avec ou sans étape de préformation. L'intensité de ce signal est identique à celle obtenue en utilisant l'amorce 3221 sans préformation.

Ces résultats montrent que la présence d'un groupement intercalant de type acridine à l'extrémité 5' de l'amorce étudiée permet la formation de la structure tige-boucle par repliement intramoléculaire, au sein d'un long hétéroduplex ARN:ADN préformé. En l'absence d'acridine, la forme duplex ARN:ADN associée à la tige-boucle correspondante semble majoritaire, puisqu'aucun signal de déplacement n'est observé. Ces résultats montrent l'intérêt que peut présenter la présence d'un agent intercalant sur les amorces pour favoriser le repliement intramoléculaire des structures tige-boucle.

### EXEMPLE 9 :

Des produits d'amplification par PCR ont été synthétisés dans les conditions décrites précédemment, à l'aide de l'amorce 1863 (SEQ ID n° : 39) et d'une des amorces suivantes : l'amorce tige-boucle 3514 (SEQ ID n° : 37) possédant un groupement acridine en 5' et un bras non nucléotidique Spacer hexaéthylène glycol (Clontech ref 5260), ou l'amorce tige-boucle 3239 (SEQ ID n° : 34) contenant un bras espaceur phosphoramidite, mais pas de groupement acridine, ou l'amorce témoin 3215 (SEQ ID n° : 43) contenant une séquence de tige mais pas de structure tige-boucle. Les produits d'amplification sont purifiées comme décrit précédemment.

Des essais de déplacement de la structure tige-boucle ont été réalisés, selon le principe décrit dans la figure 8. 10¹¹ copies de cible PCR purifiées sont mélangées à 37°C avec le tampon commercial du fragment de Klenow de l'ADN polymérase I d'*Escherichia coli,* en présence de dNTPs (1 mM) et de l'amorce de déplacement Dis 9 (SEQ ID n° : 45), dont la séquence est identique à celle de la tige de la structure tige-boucle. Cet oligonucléotide est marqué en 5' au [γ-³²P] ATP. Le volume final réactionnel est de 20 µl.

Les essais sont réalisées avec ou sans dénaturation thermique initiale (3 minutes à 95°C). 5 U de fragment de Klenow de l'ADN polymérase I d'*Escherichia coli* sont ajoutées. Après 1 heure à 37°C, les réactions sont arrêtées par congélation et les produits d'élongation sont analysés par électrophorèse en gel de polyacrylamide dénaturant. Les produits sont révélés par autoradiographie.

Les résultats révèlent des produits d'élongation d'une taille attendue de 141 bases en présence des matrices d'amplification PCR synthétisées à partir des amorces 3215, 3239 et 3516, lorsque les essais sont réalisés après dénaturation thermique. Sans étape de dénaturation thermique de la cible au préalable, un produit d'élongation de taille attendue est obtenu pour les matrices d'amplification PCR synthétisées avec les amorces tige-boucle 3516 et 3239, mais aucun produit n'est détecté dans le cas de la PCR effectuée avec l'amorce 3215 (témoin négatif). Ces éléments montrent que la présence d'une structure tige-boucle au sein d'un duplex ADN permet, par repliement de ladite structure, de libérer un site simple brin qui peut ensuite s'hybrider avec une amorce de déplacement. Cette amorce peut ensuite être élongée et peut déplacer le brin situé en aval renfermant une structure tige-boucle à son extrémité 5' (figure 8). En l'absence de structure tige-boucle (cas de l'amorce 3215), la forme double brin de la matrice empêche l'hybridation de toute amorce de déplacement pour l'hybridation.

### EXEMPLE 10 :

Des essais d'amplification selon la technique décrite dans la figure 9 ont été réalisés. Une cible de 83 paires de bases à été synthétisée par la technique PCR à l'aide des amorces 3215 (SEQ ID n° : 43) et 2709 (SEQ ID n° : 42), à partir du plasmide pBR322. Cette cible contient à une extrémité 5' la séquence promotrice de l'ARN polymérase du phage T7 et à l'autre extrémité 5' la séquence de la tige contenue dans l'amorce tige-boucle utilisée dans les essais d'amplification. La cible est utilisée dans des conditions de concentration décroissantes allant de 10^{10,} 10⁹, 10^{8,} 10⁷ et 10⁶ copies. Le milieu réactionnel (25 µl) contient l'amorce tige-boucle 3514 (SEQ ID n° : 37) contenant un groupement héxaéthylèneglycol ainsi qu'une acridine en 5', et l'amorce promotrice 2709 (SEQ ID n° : 42) à une concentration finale de 1µM. L'amorce de déplacement Dis22 (SEQ ID n° : 44), dont la séquence est identique à la séquence de la tige contenue dans l'amorce tige-boucle, est utilisée à 1 µM final. Le tampon de réaction utilisé est celui optimisé par Milligan *et al.* pour l'activité de l'ARN polymérase T7, additionné de glutamate de potassium 50m M, et contient des rNTPs (Pharmacia) (4m M) et des dNTPs (Pharmacia) (1 mM). Les enzymes utilisées sont 50 U de la T7 ARN polymérase (Biolabs) ou 200U de transcritase inverse Superscript II™ (GIBCO BRL). Pour chacune des dilutions de cible, trois essais ont été réalisés : un essai complet (comme décrit précédemment), un essai sans amorce de déplacement Dis22, et un essai sans enzymes. Après une incubation de deux heures à 37°C, les réactions d'amplification sont arrêtées par congélation du milieu réactionnel à -20°C. 1/5^{ème} de volume réactionnel est analysé quantitativement par capture et détection spécifique selon la méthode ELOSA. La technique de fixation de l'oligonucléotide de capture A20 (SEQ ID n° : 27), ainsi que la détection du produit d'amplification capturé est décrite dans l'exemple 5 ci-dessus. La fraction de 5 µl à analyser est ajoutée à un volume de 35 µl de polyéthylène glycol (PEG). Les acides nucléiques contenus dans cet échantillon sont dénaturés par addition de 5 µl d'hydroxyde de sodium 1 M. Chaque échantillon est déposé dans le puits d'une plaque de microtitration dans laquelle à été préalablement fixée la sonde de capture A20 (SEQ ID n° : 27). L'ajout de la sonde de détection A19 (SEQ ID n° : 28), l'incubation et la lecture de la microplaque sont réalisées conformément à la technique précedemment décrite dans l'exemple 5.

Les résultats obtenus (Figure 10) montrent que la méthode d'amplification permet de détecter un signal spécifique significatif jusqu'à une quantité de cible initiale de 10⁸ copies par essai, soit une sensibilité absolue de 2 x 10⁷ copies, puisqu'une fraction équivalente à 1/5 ème de milieu réactionnel est analysée dans ces conditions. En l'absence de l'amorce de déplacement Dis22, un signal spécifique significatif est obtenu jusqu'a une quantité de cible initiale de 10⁹ copies par essai, soit une sensibilité absolue de 2 x 10⁸ copies. Ces résultats mettent en évidence la possibilité d'obtenir une accumulation de produit d'amplification par cyclisation du procédé en présence de l'amorce de déplacement. En l'absence d'enzyme, la sensibilité de détection est de 10¹⁰ copies par essai.

### EXEMPLE 11 :

L'utilisation des amorces tige-boucle de la présente invention dans une méthode d'amplification telle que décrite dans la figure 4, a été étudiée. Des produits PCR de 91 paires de bases, comprenant à chaque extrémité des structures tige-boucle avec un bras non nucléotidique phosphoramidite, ont été utilisés comme cible d'amplification. Ils ont été synthétisés à l'aide des amorces 2810 (SEQ ID n° : 6) et 2811 (SEQ ID n° : 8). Ces mêmes amorces ont été utilisées à une concentration de 1 µM final dans les essais d'amplification; l'oligonucléotide déplaceur Dis9 utilisé (SEQ ID n° : 52) est marqué en 5' au [γ-³²P] ATP et utilisé à une concentration finale de 1µM. Les amplifications sont réalisées sur des dilutions de cible allant de 10¹¹ à 0 copies de cible par essai, en présence de 5 U de fragment Klenow de l'ADN polymérase d'*Escherichia coli* (Boehringer), dans le tampon commercial recommandé pour cette enzyme, et en présence de dNTPs 1 mM (Pharmacia). Les essais sont réalisés dans un volume total de milieu réactionnel de 50 µl et incubés 3 heures à 37°C. Un essai témoin sans enzyme a également été réalisé dans les mêmes conditions.

1/10^{ème} de volume réactionnel est analysée quantitativement par capture et détection spécifique selon la méthode ELOSA. La détection du produit d'amplification est réalisée à l'aide de l'oligonucléotide de capture A25 (SEQ ID n° : 14) et la sonde de détection A28 (SEQ ID n° : 15), selon la méthode décrite dans l'exemple 5.

L'analyse des résultats obtenus (Figure 11) montre qu'un signal d'amplification significatif est obtenu dans ces conditions expérimentales, pour des quantités de cible initiales de 10¹¹ et 10¹⁰ copies par essai, soit une sensibilité absolue de 10⁹ copies, puisq'une fraction équivalant à 1/10^{ème} du milieu réactionnel est analysée dans ces conditions. En absence d'enzyme, aucun signal significatif n'est obtenu, la cible de départ n'est même pas détectée. Compte tenu de la spécificité de détection de la méthode ELOSA, ces résultats montrent qu'une accumulation spécifique de produit s'effectue.

## Revendications

1. Oligonucléotide comprenant successivement, de son extrémité 5' vers son extrémité 3' :
- une première partie capable d'auto-appariement pour former une structure tige-boucle comprenant un segment amont et un segment aval appariés,
- et une seconde partie non susceptible d'auto-appariement, caractérisé par le fait que ladite première partie comprend au moins un agent bloquant, capable de bloquer la réplication par une polymérase dudit oligonucléotide, de façon qu'au moins une partie du segment aval soit répliquée et de façon que le segment amont ne soit répliqué ni totalement ni partiellement.

2. Oligonucléotide selon la revendication précédente, caractérisé par le fait que ledit agent bloquant est tel que la partie susceptible d'être répliquée du segment aval a une longueur d'au moins 2 nucléotides, et en particulier d'au moins 3 nucléotides.

3. Oligonucléotide selon l'une quelconque des revendications précédentes, caractérisé par le fait que l'extrémité aval du segment amont et l'extrémité amont du segment aval sont reliés par une boucle.

4. Oligonucléotide selon l'une quelconque des revendications précédentes, caractérisé par le fait que lesdits segments amont et aval contiennent chacun de 2 à 30, et en particulier de 3 à 15 nucléotides.

5. Oligonucléotide selon l'une quelconque des revendications précédentes, caractérisé par le fait que ledit agent bloquant comprend soit au moins un nucléotide modifié non recopiable par une polymérase soit un bras hydrocarboné.

6. Oligonucléotide selon l'une quelconque des revendications précédentes, caractérisé par le fait qu'il contient, entre lesdites première et deuxième parties, une troisième partie contenant la séquence sens d'un promoteur pour une ARN polymérase.

7. Oligonucléotide selon l'une quelconque des revendications 1 à 6, caractérisé par le fait que ladite deuxième partie contient de 5 à 40 nucléotides.

8. Amorce oligonucléotidique destinée à être utilisée dans un procédé d'amplification d'une séquence cible d'un acide nucléique, ladite séquence cible comprenant une séquence aval d'au moins 5 nucléotides, caractérisée par le fait qu'elle est constituée par un oligonucléotide tel que défini dans l'une quelconque des revendications précédentes, et que ladite deuxième partie contient à son extrémité 3' une séquence capable de s'hybrider avec ladite séquence aval de la cible.

9. Procédé d'amplification cyclique d'une séquence cible d'un acide nucléique, ladite séquence cible comprenant à son extrémité 5' une région amont et à son extrémité 3' une région aval, comprenant les étapes consistant à :
- obtenir un polynucléotide simple brin comprenant un premier segment correspondant à la séquence cible à amplifier, un second segment situé en amont de l'extrémité 5' du premier segment et un troisième segment situé en aval de l'extrémité 3' du premier segment,
ledit second segment dudit polynucléotide simple brin étant défini de la même façon que la première partie d'un oligonucléotide selon l'une quelconque des revendications 1 à 7,
et ledit troisième segment dudit polynucléotide simple brin ayant une séquence quelconque,
- mettre ledit polynucléotide en présence de :
(a) une première amorce telle que définie dans la revendication 8, capable de s'hybrider avec la région aval de la séquence cible, étant entendu qu'au moins la partie aval du segment aval de la première partie de la première amorce est complémentaire du troisième segment du polynucléotide simple brin,
(b) éventuellement une seconde amorce telle que définie dans la revendication 8, capable de s'hybrider avec une région aval de la séquence complémentaire de la séquence cible,
cette région aval étant complémentaire de ladite région amont de la séquence cible, étant entendu que la première partie de la seconde amorce est celle dont le second segment dudit polynucléotide simple brin est l'homologue,
(c) une troisième amorce dont la séquence est homologue d'au moins une partie aval de la séquence du segment aval de ladite première partie de la première amorce,
(d) une quatrième amorce dont la séquence est homologue d'au moins une partie de la séquence du segment aval de ladite première partie constituant ledit second segment dudit polynucléotide simple brin, et (e) un système enzymatique contenant au moins une activité de réplication dudit acide nucléique et une activité de déplacement de brin,
- et faire incuber le mélange obtenu dans des conditions permettant l'hybridation et le fonctionnement desdites activités enzymatiques.

10. Procédé selon la revendication 9, caractérisé par le fait que ladite première amorce est telle que définie dans la revendication 6, que ledit polynucléotide simple brin contient, entre son premier segment et son troisième segment, un quatrième segment complémentaire de ladite séquence sens contenue dans la première amorce, et par le fait que ledit système enzymatique contient en outre une activité d'ARN polymérase sous la dépendance dudit promoteur.

11. Procédé selon la revendication 9 ou 10, caractérisé par le fait que la seconde amorce est présente et est telle que définie dans la revendication 6,
que ledit polynucléotide simple brin contient, entre son premier segment et son deuxième segment, un segment supplémentaire dont la séquence est celle de la séquence sens contenue dans la deuxième amorce,
et que ledit système contient en outre une activité d'ARN polymérase sous la dépendance dudit promoteur correspondant à cette séquence sens contenue dans la deuxième amorce.

## Patentansprüche

1. Oligonucleotid, umfassend von seinem 5'-Ende gegen sein 3'-Ende folgend:
- einen zur Autohybridisierung fähigen ersten Teil zur Bildung einer Stem-Loop-Struktur (Haarnadelschleife), umfassend ein stromaufwärts und ein stromabwärts gepaartes Segment, und
- einen zweiten Teil, der nicht zur Autohybridisierung befähigt ist,
dadurch gekennzeichnet, daß das erste Teil mindestens ein Blockierungsmittel umfaßt, das fähig ist, die Replikation des Oligonucleotids durch eine Polymerase auf die Weise zu blockieren, daß mindestens ein Teil des stromabwärtigen Segments repliziert wird und das stromaufwärtige Segment weder vollständig noch teilweise repliziert wird.

2. Oligonucleotid nach dem vorhergehenden Anspruch, dadurch gekennzeichnet, daß das Blockierungsmittel so beschaffen ist, daß der replizierbare Teil des stromabwärtigen Segments eine Länge von mindestens zwei Nucleotiden, im besonderen von mindestens drei Nucleotiden hat.

3. Oligonucleotid nach einem der vorhergehenden Ansprüche, dadurch gekennzeichnet, daß das stromabwärtige Ende des stromaufwärtigen Segments und das stromaufwärtige Ende des stromabwärtigen Segments durch eine Schleife verbunden sind.

4. Oligonucleotid nach einem der vorhergehenden Ansprüche, dadurch gekennzeichnet, daß die stromaufwärtigen und stromabwärtigen Segmente je 2 bis 30, im besonderen 3 bis 15 Nucleotide enthalten.

5. Oligonucleotid nach einem der vorhergehenden Ansprüche, dadurch gekennzeichnet, daß das Blockierungsmittel entweder mindestens ein modifiziertes, durch eine Polymerase nicht kopierbares/transkribierbares Nucleotid oder einen Kohlenwasserstoffzweig umfaßt.

6. Oligonucleotid nach einem der vorhergehenden Ansprüche, dadurch gekennzeichnet, daß es zwischen dem ersten und zweiten Teil einen dritten Teil aufweist, der eine Senssequenz für eine RNS-Polymerase aufweist.

7. Oligonucleotid nach einem der Ansprüche 1 bis 6, dadurch gekennzeichnet, daß der zweite Teil 5 bis 40 Nucleotide aufweist.

8. Oligonucleotid-Primer zur Verwendung in einem Amplikationsverfahren einer Zielsequenz einer Nucleinsäure, wobei die Zielsequenz eine stromabwärtige Sequenz von mindestens von 5 Nucleotiden aufweist, dadurch gekennzeichnet, daß sie durch ein Oligonucleotid, wie in einem der vorhergehenden Ansprüche definiert, gebildet wird und daß der zweite Teil an seinem 3'-Ende eine Sequenz aufweist, die mit der stromabwärtigen Zielsequenz hybridisieren kann.

9. Cyclisches Amplifikationsverfähren der Zielsequenz einer Nucleinsäure, wobei die Zielsequenz an ihrem 5'-Ende eine Region stromaufwärts und an ihrem 3'-Ende eine Region stromabwärts aufweist, umfassend folgende Stufen:
- Erhalt eines einzelsträngigen Polynucleotids umfassend ein erstes Segment entsprechend der zu amplifizierenden Zielsequenz, ein zweites Segment oberhalb des 5'-Endes des ersten Segments und ein drittes Segment unterhalb des 3'-Endes des ersten Segments,
wobei das zweite Segment des einzelsträngigen Polynucleotids auf gleiche Weise definiert ist wie der erste Teil eines Oligonucleotids gemäß einem der Ansprüche 1 bis 7,
und wobei das dritte Segment des einzelsträngigen Polynucleotids eine beliebige Sequenz aufweist,
- Belassen des Polynucleotids in Gegenwart von:
(a) einem ersten Primer, wie in Anspruch 8 definiert, der mit einer stromabwärtigen Region der Zielsequenz hybridisieren kann, wobei mindestens der abwärtige Teil des abwärtigen Segments des ersten Teils des ersten Primers dem dritten Segment des einzelsträngigen Polynucleotids komplementär ist,
(b) gegebenenfalls einem zweiten Primer, wie in Anspruch 8 definiert, der mit einer stromabwärtigen Region der zur Zielsequenz komplementären Sequenz hybridisieren kann,
wobei diese stromabwärtige Region zu der aufwärtigen Region der Zielsequenz komplementär ist,
wobei der erste Teil des zweiten Primers der ist, der dem zweiten Segment des einzelsträngigen Polynucleotids homolog ist,
(c) einen dritten Primer, dessen Sequenz homolog zu mindestens einem Teil der Sequenz des abwärtigen Segments des ersten Teils des ersten Primers ist,
(d) einen vierten Primer, dessen Sequenz homolog zu mindestens einem Teil der Sequenz des abwärtigen Segments des ersten Teils ist, der das zweite Segment des einzelsträngigen Polynucleotids bildet, und
(e) ein enzymatisches System, enthaltend mindestens eine Replikationsaktivität der Nucleinsäure und eine Umlagerungsaktivität des Stranges,
- und Inkubieren des erhaltenen Gemisches unter Bedingungen, die die Hybridisierung und das Funktionieren der enzymatischen Aktivität erlauben.

10. Verfahren gemäß Anspruch 9, dadurch gekennzeichnet, daß der erste Primer wie in Anspruch 6 definiert ist, daß das einzelsträngige Polynucleotid zwischen seinem ersten Segment und seinem dritten Segment ein viertes Segment aufweist, das zu der im ersten Primer enthaltenen Sequenz komplementär ist und dadurch, daß das enzymatische System außerdem eine RNS-Polymeraseaktivität unter Kontrolle des Promotors aufweist.

11. Verfahren nach Anspruch 9 oder 10, dadurch gekennzeichnet, daß der zweite Primer anwesend und wie in Anspruch 6 definiert ist, daß das einzelsträngige Polynucleotid zwischen seinem ersten Segment und seinem zweiten Segment ein zusätzliches Segment enthält, dessen Sequenz der Sequenz entspricht, die im zweiten Primer enthalten ist, und daß das System außerdem eine RNS-Polymeraseaktivität aufweist, abhängig vom Promotor, entsprechend der im zweiten Primer enthaltenen Senssequenz.

## Claims

1. Oligonucleotide comprising successively, from its 5' end to its 3' end,
- a first portion capable of self-pairing in order to form a pin-loop structure comprising an upstream segment and a downstream segment which are paired,
- and a second portion not capable of self-pairing, characterized in that the said first portion comprises at least one blocking agent capable of blocking the replication, by a polymerase, of the said oligonucleotide, such that at least a portion of the downstream segment is replicated and such that the upstream segment is replicated neither totally nor partially.

2. Oligonucleotide according to the preceding claim, characterized in that the blocking agent is such that the portion of the downstream segment capable of being replicated has a length of at least 2 nucleotides and in particular of at least 3 nucleotides.

3. Oligonucleotide according to any one of the preceding claims, characterized in that the downstream end of the upstream segment and the upstream end of the downstream segment are linked by a loop.

4. Oligonucleotide according to any one of the preceding claims, characterized in that the said upstream and downstream segments each contain from 2 to 30, and in particular from 3 to 15 nucleotides.

5. Oligonucleotide according to any one of the preceding claims, characterized in that the said blocking agent comprises either at least one modified nucleotide non-recopiable by a polymerase, or one hydrocarbon arm.

6. Oligonucleotide according to any one of the preceding claims, characterized in that it contains, between the said first and second portions, a third portion containing the sense sequence of a promoter for an RNA polymerase.

7. Oligonucleotide according to any one of Claims 1 to 6, characterized in that the said second portion contains from 5 to 40 nucleotides.

8. Oligonucleotide primer intended to be used in a process for the amplification of a target sequence of a nucleic acid, the said target sequence comprising a downstream sequence of at least 5 nucleotides, characterized in that it consists of an oligonucleotide as defined in any one of the preceding claims, and in that the said second portion contains, at its 3' end, a sequence capable of hybridizing with the said downstream sequence of the target.

9. Process for the cyclic amplification of a target sequence of a nucleic acid, the said target sequence comprising at its 5' end an upstream region and at its 3' and a downstream region, comprising the stages consisting of:
- obtaining a single-stranded polynucleotide comprising a first segment corresponding to the target sequence to be amplified, a second segment situated upstream of the 5' end of the first segment and a third segment situated downstream of the 3' end of the first segment,
the said second segment of the said single-stranded polynucleotide being defined in the same way as the first portion of an oligonucleotide according to any one of claims 1 to 7,
and the said third segment of the said single-stranded polynucleotide having any sequence,
- exposing the said polynucleotide to:
(a) a first primer as defined in Claim 8, capable of hybridizing with the downstream region of the target sequence, it being understood that at least the down-stream portion of the downstream segment of the first portion of the first primer is complementary to the third segment of the single-stranded polynucleotide,
(b) optionally a second primer as defined in Claim 8, capable of hybridizing with a downstream region of the sequence complementary to the target sequence,
this downstream region being complementary to the said upstream region of the target sequence,
it being understood that the first portion of the second primer is that of which the second segment of the said single-stranded polynucleotide is the homologue,
(c) a third primer whose sequence is homologous to at least a downstream portion of the sequence of the downstream segment of the said first portion of the first primer,
(d) a fourth primer whose sequence is homologous to at least, a portion of the sequence of the downstream segment of the said first portion constituting the said second segment of the said single-stranded polynucleotide,
and (e) an enzymatic system containing at least an activity for replicating the said nucleic acid and a strand displacement activity,
- and incubating the mixture obtained under conditions allowing the hybridization and the functioning of the said enzymatic activities.

10. Process according to Claim 9, characterized in that the said first primer is as defined in Claim 6, in that the said single-stranded polynucleotide contains, between its first segment and its third segment, a fourth segment complementary to the said sense sequence contained in the first primer, and in that the said enzymatic system contains, in addition, an RNA polymerase activity under the control of the said promoter.

11. Process according to Claim 9 or 10, characterized in that the second primer is present and is as defined in Claim 6, in that the said single-stranded polynucleotide contains, between its first segment and its second segment, an additional segment whose sequence is that of the sense sequence contained in the second primer,
and in that the said system contains, in addition, an RNA polymerase activity under the control of the said promoter corresponding to this sense sequence contained in the second primer.
